(19) Europäisches Patentamt
European Patent Office
Office européen des brevets

(11) **EP 4 012 397 B1**

(12) **EUROPÄISCHE PATENTSCHRIFT**

(45) Veröffentlichungstag und Bekanntmachung des Hinweises auf die Patenterteilung:
**08.03.2023   Patentblatt 2023/10**

(21) Anmeldenummer: **21197496.9**

(22) Anmeldetag: **17.09.2021**

(51) Internationale Patentklassifikation (IPC):
*G01N 27/622* (2021.01)     *G01N 27/68* (2006.01)
*H05H 1/24* (2006.01)

(52) Gemeinsame Patentklassifikation (CPC):
**G01N 27/622; G01N 27/68; H05H 1/2406**

(54) **IONENMOBILITÄTSSPREKTROMETER UND VERFAHREN ZUM BETRIEB EINES IONENMOBILITÄTSSPEKTROMETERS**

ION MOBILITY SPECTROMETER AND METHOD FOR OPERATING SAME

SPECTROMÈTRE DE MOBILITÉ IONIQUE ET PROCÉDÉ DE FONCTIONNEMENT D'UN SPECTROMÈTRE DE MOBILITÉ IONIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.12.2020   DE 102020132851**

(43) Veröffentlichungstag der Anmeldung:
**15.06.2022   Patentblatt 2022/24**

(73) Patentinhaber: **Bruker Optics GmbH & Co. KG**
**76275 Ettlingen (DE)**

(72) Erfinder:
• **Beyer, Achim**
**04821 Brandis (DE)**
• **Renner, Uwe**
**04105 Leipzig (DE)**
• **Heller, Wolfgang**
**04319 Leipzig (DE)**
• **Faust, Sascha**
**04105 Leipzig (DE)**

(74) Vertreter: **Gulde & Partner**
**Patent- und Rechtsanwaltskanzlei mbB**
**Wallstraße 58/59**
**10179 Berlin (DE)**

(56) Entgegenhaltungen:
**EP-A1- 1 963 835     GB-A- 2 584 334**
**US-B2- 9 953 818**

• **JING HU ET AL: "Dielectric Barrier Discharge in Analytical Spectrometry", APPLIED SPECTROSCOPY REVIEWS, Bd. 46, Nr. 5, 1. Juli 2011 (2011-07-01), Seiten 368-387, XP055208260, ISSN: 0570-4928, DOI: 10.1080/05704928.2011.561511**

## Beschreibung

Die vorliegende Erfindung betrifft ein lonenmobilitätsspektrometer, insbesondere ein mobiles lonenmobilitätsspektrometer mit verbesserter Plasmazündung, sowie ein Verfahren zum Betrieb eines lonenmobilitätsspektrometers, insbesondere mit minimaler Zündspannung.

## TECHNISCHER HINTERGRUND

Ein lonenmobilitätsspektrometer ist ein Instrument zur Charakterisierung von Ionen anhand ihrer Mobilität in einem Driftmedium unter der Wirkung einer elektromagnetischen Kraft. Ein lonenmobilitätsspektrometer weist in der Regel eine Reaktionskammer sowie einen daran angrenzenden Driftraum auf. Dabei dient die Reaktionskammer der Bildung von Reaktant-Ionen sowie der Ionisierung des Probenmaterials. Der Driftraum dient dem Bestimmen der Flug- bzw. Driftzeit der gebildeten Ionen.

Die Probe gelangt über Einlässe und mittels eines Trägergases in den Reaktionsraum und wird dort durch Energiezufuhr ionisiert. Die gebildeten Ionen werden durch elektrische Feldeinwirkung in den Driftraum geleitet, der beispielsweise eintrittsseitig durch ein Gitter als Ionenmodulator und gegenüberliegend durch eine Detektoreinheit zur Ionenstrommessung begrenzt ist. In dem Driftraum werden die Ionen durch weitere Feldeinwirkung in Richtung des Detektors bewegt. Ein im Driftraum enthaltenes Driftmedium mit definierten physikalisch-chemischen Eigenschaften wechselwirkt mit den Ionen und wirkt der durch die Feldeinwirkung vermittelten Bewegung der Ionen entgegen. Der Transport der Ionen im Driftraum hängt somit von der elektrischen Feldstärke als auch von der Wechselwirkung mit dem Driftgas ab und ist durch die resultierende Driftgeschwindigkeit charakterisierbar.

Die lonenmobilität K ist somit ein Maß für die molekulare Reibung eines Ions in einem bestimmten Driftmedium unter der Wirkung einer elektromagnetischen Kraft und ist umso größer, je kleiner diese Reibung ist. Bestimmt wird die lonenmobilität K als Proportionalitätsfaktor zwischen der Driftgeschwindigkeit v und der in Driftrichtung wirksamen elektrischen Feldkomponente E. In erster Näherung gilt zwischen diesen Größen die lineare Beziehung

$$v = K*E. \qquad (1)$$

Sofern die Eigenschaften des Driftmediums im Driftraum homogen vorliegen und das elektrische Feld im Driftbereich der Ionen konstant ist, bestimmt sich die lonenmobilität auf einer vorgegebenen Wegstrecke L durch Bestimmung der Driftzeit t und der Spannungsdifferenz U zwischen Ende und Anfang der Driftstrecke anhand der aus (1) abgeleiteten Gleichung:

$$K = L^2 /(U*t). \qquad (2)$$

Die so ermittelte lonenmobilität K bezieht sich dabei stets auf ein konkretes Driftmedium und ist keine spezifische Konstante eines bestimmten Ions. Vielmehr hängt der Wert der lonenmobilität von der Wechselwirkung des Ions mit dem Driftmedium ab. Wird diese Wechselwirkung als Stoßprozess angenommen, hängt deren Stärke gemäß der kinetischen Gastheorie von der Masse der beteiligten Stoßpartner, Teilchenzahldichte, Druck und Temperatur des Driftmediums und dem Wirkungsquerschnitt des Ions mit den Molekülen des Driftgases ab.

Bei unter atmosphärischen Bedingungen eingesetzten lonenmobilitätsspektrometern erstreckt sich der Wertebereiche der Teilchenzahldichte des Driftgases, welche wiederum von dessen Temperatur T und dem Druck p abhängt, über einen großen Bereich. Beispielsweise ist für die Temperatur T eine Spanne von -30°C bis +60°C und für den Druck ein Bereich von 50 kPa bis 110 kPa möglich. Die Abhängigkeit der lonenmobilität von der Teilchenzahldichte wird daher durch Umrechnung auf eine normierte lonenmobilität eliminiert. Selbst die normierte lonenmobilität $K_0(T)$ ist jedoch aufgrund der Abhängigkeit vom temperaturabhängigen Wirkungsquerschnitt der jeweiligen Wechselwirkung temperaturabhängig.

Zum Bestimmen der temperaturabhängigen normierten lonenmobilität $K_0(T)$ sind die Messungen einer Vielzahl von Werten, wie Druck, Temperatur, Driftspannung, Driftzeit notwendig, wobei sich die relativen Messfehler der Einzelmessungen summieren können. Es ist daher vorteilhaft, stattdessen ein referenzbasiertes Verfahren zum Bestimmen der lonenmobilität anzuwenden. Dabei wird eine Referenzsubstanz verwendet, für welche eine normierte, temperaturabhängige lonenmobilität $K_0^R(T)$ bekannt ist. Sofern die physikalisch-chemischen Bedingungen im Driftraum bei Referenz- und Probenmessung gleich sind, ist durch Vergleich der Driftzeiten der Referenzsubstanz und der Probensubstanz die lonenmobilität der Probensubstanz $K_0^P(T)$ mit der Driftzeit $t_P$ aus dem Driftzeitverhältnis $t_R / t_P$ durch $K_0^P(T) = t_R / t_P * K_0^R(T)$ berechenbar. Vorteilhaft sind bei diesem Verfahren lediglich der Messwertfehler der Driftzeitmessung (welcher bei ausreichend hoher Messwertabtastung im Bereich des Rauschens liegt) sowie der Messfehler der Driftraumtemperatur T (welcher nur bei einer stark von T abhängigen Ionenmobilität bedeutsam wird) relevant. Die Referenzsubstanz wird dabei in der Regel extern zugeführt, entweder über die Probenzuleitung oder über einen zusätzlichen Gaseinlass, der über ein Ventil zuschaltbar sein kann. Die Referenzsubstand, zum Beispiel NOx, kann jedoch auch im Ionenmobilitätsspektrometer erzeugt werden.

Beim Bestimmen der Ionenmobilität wird daher in der Regel vorausgesetzt, dass das Driftmedium homogene und konstante physikalisch-chemische Eigenschaften besitzt. Verwendet man jedoch beispielsweise Luft als Driftmedium, so kann bereits der darin vorhandene Anteil an Wasser den Wert der Ionenmobilität signifikant beeinflussen. Daher sollte trockene Luft, vorzugsweise mit einer absoluten Wasserkonzentration kleiner 100 ppm als Driftmedium verwendet werden. Hierzu werden in der Regel Filtersysteme eingesetzt.

Damit das Driftmedium seine definierten physikalisch-chemischen Eigenschaften beibehält, wird es in dem Driftraum zudem stetig durch einen Gasfluss erneuert. Mit anderen Worten wird der Driftraum durch einen kontinuierlichen Driftgasfluss durchströmt, wobei der Gasfluss durch Integration der Gasflussdichte über die vom Gas durchströmte Querschnittsfläche bestimmt ist. Die Gasflussdichte ist dabei das Produkt aus Gaskonzentration und Gasgeschwindigkeit. Der Gasfluss im Driftraum ist dem Ionenfluss überlagert und kann die effektive Driftzeit prinzipiell beeinflussen. Die Driftgasgeschwindigkeit von beispielsweise 4 cm/s ist jedoch im Vergleich zur Driftgeschwindigkeit der Ionen von beispielsweise 4 m/s deutlich kleiner und daher für das Bestimmen der Ionenmobilität oft vernachlässigbar.

Die Ionisierung der zu analysierenden Probe erfolgt in einem Ionenmobilitätsspektrometer zunächst über die Zufuhr von Energie mittels einer Ionisierungsquelle. Dem Fachmann sind eine Vielzahl von Ionisierungsquellen bekannt, beispielsweise Plasmaquellen. Aus den mittels der Quelle gebildeten Primär-Ionen können durch komplexe Molekül-Ionen-Reaktionen und Ladungstransferprozesse mit der Probensubstanz weitere Produkt-Ionen entstehen. Aufgrund des mehrstufigen Charakters der Ionisierung findet diese im Reaktionsraum statt, der durch den Bereich zwischen Ionisierungsquelle und Ionenstrommodulator gebildet wird.

Eine effiziente Ionisierung an der Ionisierungsquelle ist Voraussetzung für nachfolgende Ionisierungen sowie für einen effizienten Messbetrieb. Zwar steigt je nach Anregungsart mit höheren Energien, Intensitäten beziehungsweise Leistungen der Ionisierungsquelle in der Regel die Menge erzeugter Primär-Ionen, nachteilig ist jedoch auch die Menge der gebildeten NOx-Verbindungen und des gebildeten Ozons. Dabei sind Ozon, Stickoxide und Stickoxidverbindungen (NOx-Verbindungen) für den Einsatz im Ionenmobilitätsspektrometer nachteilig, da diese die Ionisierung bestimmter Probenmoleküle hemmen oder verhindern. Beispielsweise binden NOx-Verbindungen aufgrund ihrer hohen Elektronenaffinität negative Ladungen, welche damit für weitere Ionisierung der Primär-Ionen nicht zur Verfügung stehen. Die vermehrte Bildung von Ozon führt zu ungewollten Nebenreaktionen und höherem Verschleiß der sich im Gaskreislauf befindlichen Komponenten wie Pumpen und Sensoren. Somit ist der Bereich der zur Probenionisierung einsetzbaren Energie, Intensität beziehungsweise Leistung limitiert.

Aus GB2584334A, US9953818B2 und EP1963835A1 sind Ionenmobilitätsspektrometrie Anordnungen bekannt, die unter anderem einen Drucksensor und einen Temperatursensor umfassen. Die gemessenen Ionenmobilitätsspektren werden anhand der gemessenen Druck- und Temperaturwerte korrigiert.

Die Aufgabe der vorliegenden Erfindung ist es, die Nachteile des Standes der Technik zu überwinden oder zu minimieren, und ein verbessertes Ionenmobilitätsspektrometer sowie ein verbessertes Verfahren zum Betrieb eines Ionenmobilitätsspektrometers bereitzustellen.

## BESCHREIBUNG DER ERFINDUNG

Ein erster Aspekt der vorliegenden Offenbarung betrifft ein Ionenmobilitätsspektrometer (IMS) zum Ermitteln einer Ionenmobilität einer Probe, bevorzugt eines Ionenmobilitätsspektrums der Probe, bevorzugt aus einem Driftzeitspektrum gemäß Gleichung (2), insbesondere einer in Probengas (Probe und Trägergas) befindlichen Probe. Die Ionenmobilität der Probe wird vorzugsweise in trockener Luft als Driftgas ermittelt, insbesondere in Luft mit einer Wasserkonzentration von weniger als 100 ppm. Das Ionenmobilitätsspektrometer weist einen Driftraum auf, insbesondere zum Ermitteln der Ionenmobilität der ionisierten Probe in einem Driftgasstrom. Ferner weist das Ionenmobilitätsspektrometer einen Reaktionsraum auf, insbesondere zum Ionisieren des Probengases. Ein detaillierter Aufbau des Ionenmobilitätsspektrometers wird untenstehend im Detail erläutert.

Das erfindungsgemäße Ionenmobilitätsspektrometer weist eine im Reaktionsraum angeordnete dielektrisch behinderte Ionisierungsquelle (nachfolgend DBDI-Quelle genannt) zum Ionisieren des Probengases auf. Der Einsatz von DBDI-Quellen zum Erzeugen von Niedertemperatur-Plasma in einem Ionenmobilitätsspektrometer ist prinzipiell aus dem Stand der Technik bekannt. Bei einer solchen DBDI-Quelle sind vorteilhaft sämtliche Elektroden durch ein plasmaresistentes Dielektrikum geschützt und die Durchschnittstemperatur des erzeugten Niedertemperatur-Plasmas entspricht annähernd der Umgebungstemperatur. Das erzeugte Plasma ist im Mittel ladungsneutral und besteht aus einem Gemisch von negativen Ladungsträgern wie Elektronen und Ionen, positiv geladenen Ionen und Neutralteilchen.

Die Elektroden der DBDI-Quelle sind zumindest teilweise durch das Dielektrikum vom Plasma getrennt, wodurch diese vor Korrosion geschützt sind. Ein Ladungstransfer erfolgt durch diese Isolierung nicht, weswegen ein Plasma mittels Wechselspannung, insbesondere hochfrequenter Wechselspannung erzeugt wird. Das Zünden des Plasmas erfolgt unter Atmosphärenbedingungen in einer Zone von wenigen Millimetern zwischen den Elektroden.

Ausgehend von den in Luft maßgeblich vorhandenen Molekülen, wie molekularer Sauerstoff und Stickstoff, wird die Zusammensetzung des Plasmas durch komplexe chemische Reaktionen der Ionen und Neutralgasteilchen bestimmt, wobei als Nebenprodukte auch Ozon und Sauerstoffverbindungen des Stickstoffs entstehen. Die lokal begrenzt gebildeten Ionen werden durch ein dem Entladungsfeld überlagertes elektrisches Feld von der Quelle abgeführt.

Voraussetzung für die Entstehung eines Plasmas an der DBDI-Quelle sind hohe reduzierte elektrische Feldstärken, die durch Anlegen hoher Spannung an den Elektroden erreicht werden. Gemäß PASCHEN - Gesetz ist für die Entstehung des Plasmas eine Mindestzündspannung notwendig, die für Gleich- und Wechselspannung unterschiedlich sein kann. Nach dem Zünden des Plasmas kann es mit geringerer Spannung aufrechterhalten werden.

Das erfindungsgemäße Ionenmobilitätsspektrometer weist ferner einen im Reaktionsraum angeordneten Drucksensor und einen im Reaktionsraum angeordneten Temperatursensor, sowie eine mit dem Drucksensor, dem Temperatursensor und der DBDI-Quelle verbundene Steuereinheit auf. Die Steuereinheit ist erfindungsgemäß dazu eingerichtet, eine Zündspannung der DBDI-Quelle in Abhängigkeit eines durch den Drucksensor ermittelten Druckwerts und eines durch den Temperatursensor ermittelten Temperaturwerts einzustellen. Der Zündvorgang des Plasmas an einer DBDI-Quelle ist aufgrund der Abhängigkeit von der reduzierten elektrischen Feldstärke druck- und temperaturabhängig. Da bei der Plasmabildung komplexe kinetische Prozesse ablaufen, ist diese auch explizit von der Temperatur abhängig. Der Drucksensor und der Temperatursensor sind bevorzugt in der Nachbarschaft der DBDI-Quelle angeordnet, beispielsweise in einem Abstand von weniger als 5cm, besonders bevorzugt von weniger als 2cm. Ebenfalls bevorzugt sind die Sensoren in einem Gasauslass des Reaktionsraums angeordnet.

In dem erfindungsgemäßen Ionenmobilitätsspektrometer erfolgt somit das Zünden und Aufrechterhalten des Plasmas in Abhängigkeit von Temperatur und Druck des vorbeiströmenden Gases kontrolliert über die entsprechend konfigurierte Steuereinheit. Bevorzugt stellt die Steuereinheit die optimalen Feldstärke- und Hochspannungsparameter für Amplitude und Frequenz der Zündspannung ein und führt diese bei Änderung des Gaszustandes nach. Ebenfalls bevorzugt stellt die Steuereinheit die Zündspannung in Abhängigkeit der Elektrodenanordnung des speziell verwendeten Typs der DBDI-Quelle ein. Besonders bevorzugt erfolgt durch die Druck- und Temperatursensoren eine kontinuierliche (ständige) Messung der Gastemperatur und des Gasdrucks in der Umgebung der DBDI-Quelle, um das Zünden und Aufrechterhalten des Plasmas mit minimaler Zündspannung dauerhaft zu gewährleisten.

In erster Näherung ist bei einer vorgegebenen oder anhand des DBDI-Quellen-Typs (insbesondere der Elektrodenanordnung) ermittelten Zündfrequenz und bei konstantem Gasfluss die optimale Zündspannung ausschließlich von Druck und Temperatur abhängig, da diese Parameter die Gasdichte und somit die reduzierte Feldstärke bestimmen. Untersuchungen der Erfinder haben gezeigt, dass eine Einstellung der Zündspannung anhand von Druck und Temperatur ausreichend ist, um einen Betrieb mit minimaler Zündspannung sicherzustellen.

Das erfindungsgemäße Ionenmobilitätsspektrometer weist ferner eine Lichtquelle zum Bestrahlen der DBDI-Quelle mit Licht des Wellenlängenbereichs von 240nm bis 480nm auf, bevorzugt mit Licht des Wellenlängenbereichs von 280 nm bis 480 nm. Die Lichtquelle ist mithin innerhalb oder nahe des Reaktionsraums angeordnet und relativ zu der DBDI-Quelle so positioniert oder mit dieser über einen Lichtleiter verbunden, dass die DBDI-Quelle mit dem von der Lichtquelle emittierten Licht beleuchtet wird. Die Steuereinheit des Ionenmobilitätsspektrometers ist ferner mit der Lichtquelle verbunden und dazu eingerichtet, die Lichtquelle anzusteuern. Insbesondere ist die Steuereinheit des Ionenmobilitätsspektrometers dazu eingerichtet, die Lichtquelle zu aktivieren und zu deaktivieren. Bevorzugt ist die Lichtquelle dazu eingerichtet, die Intensität variabel einzustellen.

Wie bereits dargestellt, ist das Zünden des Plasmas in der Einschaltphase ein kritischer Vorgang. Wie von den Erfindern gefunden, kann das Zünden des Plasmas bei trockener, sauberer Luft (insbesondere mit einer Wasserkonzentration von weniger 100ppm) nach längerem Nichtbetrieb zeitlich stark verzögert einsetzen. Dies ist insbesondere für ein mobiles Gerät mit geforderter schneller Einsatzbereitschaft unakzeptabel. Prinzipiell könnte in solch einem Fall die Zündspannung stark erhöht werden, wodurch aber massiv störende NOx-Verbindungen gebildet würden. Hinzu kommt noch eine deutlich stärke (Über-)Belastung des Dielektrikums der DBDI-Quelle mit drohendem vorzeitigem Ausfall bzw. hohe Überschlagswahrscheinlichkeit im Elektrodenbereich wegen der zunächst sehr großen Plasmazone bei Einsetzen der Zündung mit erhöhter Spannung. Es ist prinzipiell bekannt, dass Licht zur Verringerung der Zündspannung eines Plasmas eingesetzt werden kann. Versuche der Erfinder haben jedoch gezeigt, dass beispielsweise grünes oder rotes Licht mit geringer Strahlungsleistung kleiner 1 W keinerlei Verbesserung des Zündverhaltens bewirkt. Experimentell wurde ferner gefunden, dass sich für trockene Luft die Zeit zum Zünden durch blaues bis ultraviolettes Licht, insbesondere im Wellenlängenbereich 240nm bis 480nm, besonders bevorzugt im Wellenlängenbereich 280nm bis 480nm, signifikant verkürzen lässt. Dieser Wellenlängenbereich entspricht der zweiten positiven Serie molekularen Stickstoffs. Prinzipiell können auch kürzere Wellenlängen als 240nm zur Verringerung der Zündspannung verwendet werden, doch steigt damit die Bildungsrate von Ozon und von NOx-Verbindungen deutlich an.

Die Kontrolle der Lichtquelle durch die Steuereinheit des erfindungsgemäßen Ionenmobilitätsspektrometers ermöglicht somit vorteilhaft eine bedarfsgerechte zusätzliche Reduktion der notwendigen Zündspannung, insbesondere bei erneuter Inbetriebnahme. Dies ist insbesondere auch hinsichtlich der im Vergleich zur mittleren Einsatzdauer eines lonenmobilitätsspektrometers (bis zu 10000 Stunden ohne Wartung) kürzeren Lebensdauer typischer Lichtquellen (typischerweise unter 1000 Stunden) von besonderem Vorteil. Bei der Lichtquelle handelt es sich bevorzugt um eine Leucht- oder Laserdiode mit entsprechender Wellenlänge.

In einer bevorzugten Ausführungsform des erfindungsgemäßen lonenmobilitätsspektrometers weist dieses ferner einen in dem Driftraum angeordneten lonendetektor auf. Gemäß dieser Ausführungsform ist die Steuereinheit ferner dazu eingerichtet, einen Signalwert von dem lonendetektor zu empfangen. Der lonendetektor ist bevorzugt, gegebenenfalls in Kombination mit einem unten erläuterten Schaltgitter, dazu ausgebildet, an einer Detektoreinheit ankommende lonen zu detektieren. Die ankommenden lonen sind dabei durch die Driftzeit im Driftraum, insbesondere vom Schaltgitter zum lonendetektor, und gegebenenfalls anhand der durch die lonen transportierten Ladung identifizierbar. Ein Signalwert gemäß dieser Ausführungsform korrespondiert daher bevorzugt zu einer lonenspezies, beispielsweise zu Produkt-lonen oder NOx-lonen, sowie zu einer Quantität dieser lonen-Spezies. Anhand des von dem lonendetektor erhaltenen Signalwerts kann die Steuereinheit somit insbesondere die Art und/oder die Menge der im Reaktionsraum gebildeten lonen ermitteln. Gemäß dieser Ausführungsform ist die Steuereinheit ferner dazu ausgebildet, die DBDI-Quelle und/oder die Lichtquelle anhand des von dem lonendetektor erhaltenen Signalwerts anzusteuern. Dies ermöglicht bevorzugt eine Regelung von der DBDI-Quelle und/oder der Lichtquelle im Hinblick auf das gezielte Erzeugen und/oder gezielte Vermeiden bestimmter lonenspezies.

Besonders bevorzugt ist die Steuereinheit des erfindungsgemäßen lonenmobilitätsspektrometers gemäß dieser Ausführungsform dazu eingerichtet, die Lichtquelle so anzusteuern, dass ein erster Signalwert des lonendetektors einen ersten Grenzwert überschreitet. In einer bevorzugten Durchführungsform ist der erste Signalwert ein über einen größeren Driftzeitbereich, insbesondere den gesamten zu erfassenden Driftzeitbereich, berechneter Integral- oder Summenwert, der aus einem mit geöffnetem Gitter aufgenommen Driftzeitspektrum bestimmt wird und ein Maß für die durch die DBDI-Quelle erzeugte Ladung darstellt. Alternativ wird von dem lonendetektor zunächst ein erster Signalwert ausgewertet, dessen Lage im lonenmobilitätsspektrum bevorzugt zu einer bestimmten lonenspezies korrespondiert. Diese lonenspezies ist bevorzugt vorbestimmt und entspricht besonders bevorzugt einem zu detektierenden oder erwarteten Produkt-lon. Alternativ kann es sich jedoch auch um eine beliebige andere lonen-Spezies handeln. Ferner bevorzugt korrespondiert der erste Signalwert zu einem unspezifischen Integral des Spektrums oder zu einem nicht modulierten Gesamtionenstrom, um das Zünden eines Plasmas zu erkennen.

Gemäß dieser Ausführungsform ist der erste Signalwert bevorzugt proportional der Menge der im Reaktionsraum erzeugten lonen der jeweiligen Spezies. Dies ist insbesondere bei kleinen Amplituden im Driftspektrum der zutreffend. Bevorzugt ist die Steuereinheit gemäß dieser Ausführungsform dazu ausgebildet, ein Unterschreiten eines vorbestimmten Grenzwerts durch den ersten Signalwert zu ermitteln. Dies kann darauf hindeuten, dass ein Zünden des Plasmas nicht erfolgreich war oder dass das Plasma nicht konstant aufrechterhalten werden kann. Die Steuereinheit dieser Ausführungsform ist ferner bevorzugt dazu ausgebildet, in Reaktion auf das Ermitteln des Unterschreitens des vorbestimmten Grenzwerts durch den ersten Signalwert, die Lichtquelle zum Bestrahlen der DBDI-Quelle mit Licht einer Wellenlänge von 240nm bis 480nm, bevorzugt 280 nm bis 480 nm, anzusteuern. Somit kann das Zünden des Plasmas beziehungsweise dessen Aufrechterhaltung durch die Steuereinheit unterstützt werden. Ebenfalls bevorzugt empfängt die Steuereinheit eine Mehrzahl von Signalwerten, beispielsweise zu unterschiedlichen lonenspezies, und führt einen Vergleich dieser Signalwerte mit einem oder mehreren Grenzwerten durch, um zu ermitteln ob ein Beleuchten der DBDI-Quelle angezeigt oder notwendig ist und steuert die Lichtquelle schließlich basierend auf dem Ergebnis dieser Vergleiche an.

In einer ebenfalls besonders bevorzugten Ausführungsform des erfindungsgemäßen lonenmobilitätsspektrometers ist die Steuereinheit ferner dazu eingerichtet, eine Zündspannung der DPBI-Quelle so einzustellen, dass bei den ermittelten Druck- und Temperaturwerten ein erster Signalwert eines lonendetektors einen ersten Grenzwert überschreitet und ein zweiter Signalwert des lonendetektors einen zweiten Grenzwert unterschreitet. Besonders bevorzugt empfängt die Steuereinheit gemäß dieser Ausführungsform einen zu einer ersten lonen-Spezies korrespondierenden ersten Signalwert sowie einen zu einer zweiten lonen-Spezies korrespondierenden zweiten Signalwert. Die Steuereinheit kann dann ermitteln, ob der erste Signalwert einen ersten Grenzwert überschreitet und ob der zweite Signalwert einen zweiten Grenzwert unterschreitet und kann anhand dieser Vergleiche die DBDI-Quelle ansteuern.

Besonders bevorzugt ist die Steuereinheit gemäß dieser Ausführungsform dazu eingerichtet, den ersten Signalwert anhand eines Peaks eines Produkt-lons und den zweiten Signalwert anhand eines Peaks eines NOx-lons in einem mit dem lonendetektor ermittelten lonenmobilitätsspektrum zu ermitteln. Mit anderen Worten empfängt die Steuereinheit in diesem Fall ein von dem lonendetektor ermitteltes lonenmobilitätsspektrum beziehungsweise erstellt ein solches Spektrum anhand vom lonendetektor empfangener Daten. Ferner bevorzugt identifiziert die Steuereinheit anhand vordefinierter Informationen (beispielsweise anhand einer Nutzereingabe und/oder eines Speicherwerts) einen Peak eines Produkt-lons und einen Peak eines NOx-lons in dem lonenmobilitätsspektrum und liest die zu den jeweiligen Peak-Lagen korrespondierenden Signalwerte aus dem lonenmobilitätsspektrum aus. Mithin korrespondiert das Überschreiten eines ersten Grenzwerts durch den ersten Signalwert zum Vorliegen von mehr als einer bestimmten Menge an Produkt-Ionen im Driftraum und korrespondiert das Unterschreiten eines zweiten Grenzwerts durch den zweiten Signalwert das Vorliegen von weniger als einer bestimmten Menge an NOx-Ionen im Driftraum.

Die vorgenannten bevorzugten Ausführungsformen ermöglichen einen vorteilhaften Betrieb des Ionenmobilitätsspektrometers mit einer minimalen Zündspannung mit der eine ausreichende Produktion von Produkt-Ionen und eine minimale Verschmutzung durch NOx-Ionen gewährleistet wird. Wie vorstehend erläutert, liegt eine optimale Zündspannung vor, wenn zuverlässig ein Mindestmaß an Produkt-Ionen (Primär-Ionen, Proben-Ionen), aufrechterhalten werden kann und die Produktion von NOx-Ionen minimiert wird. Diese optimale Zündspannung wird bevorzugt anhand eines Ionenmobilitätsspektrums ermittelt, insbesondere unter Berücksichtigung der jeweiligen Peak-Lagen. Besonders bevorzugt ist die Vermeidung der Bildung sämtlicher NOx-Ionen, d.h. das Minimieren der Einzelintensitäten oder von Integralen (Summen) der Intensitäten in einem Intervall um die NOx-Peak-Lagen auf null. Ebenso bevorzugt ist das Bilden einer ausreichenden Menge Produkt-Ionen (Primär-Ionen, Proben-Ionen), das heißt das Gewährleisten von Einzelintensitäten oder von Integralen (Summen) der Intensitäten in einem Intervall um die jeweilige Peak-Lagen oberhalb eines Grenzwerts.

Das Ermitteln der optimalen Zündspannung ist somit ein mehrdimensionales Optimierungsproblem, welches bevorzugt rechentechnisch unter Berücksichtigung vorbestimmter (erster und zweiter) Grenzwerte ausgeführt wird. Besonders bevorzugt wird das Optimierungsproblem von der erfindungsgemäßen Steuereinheit anhand vom Ionendetektor empfangener Daten gelöst. Ebenfalls bevorzugt wird dieses Optimierungsproblem anhand von in einem Teststand ermittelter Daten gelöst. Besonders bevorzugt wird dieses Optimierungsproblem unter Verwendung mathematischer Algorithmen, wie der Methode der kleinsten Quadrate oder mittels künstlicher Intelligenz und Parametervariation gelöst. Besonders bevorzugt wird die Optimierungsaufgabe für eine bestimmte DBDI-Quelle gelöst, indem unter definierten thermodynamischen Bedingungen in einem Teststand, beispielsweise einem Klimaschrank, durch Variation von Druck und Temperatur und gegebenenfalls der Frequenz, die minimale Zündspannung bestimmt wird, während die DBDI-Quelle mit trockener, sauberer Luft mit einer Wasserkonzentration kleiner 100 ppm bei definiertem Gasfluss umströmt wird.

Als Ergebnis der Optimierung wird erfindungsgemäß für eine bestimmte Zündfrequenz eine Druck-Temperatur-Matrix-Tabelle erhalten, welche als Ergebnis die gewünschte minimale Zündspannung enthält. Durch Interpolation der Daten lässt sich ein funktionaler Zusammenhang darstellen. Die gewonnenen Daten (Tabelle) beziehungsweise Zusammenhänge sind bevorzugt auf herkömmlicher Weise in nichtflüchtigen Speichern abgelegt oder als Programme in Algorithmen gespeichert. Ferner bevorzugt weist das erfindungsgemäße Ionenmobilitätsspektrometer die gewonnen Daten in Form einer in einem internen Speicher abgelegten Lookup-Tabelle auf. Die Steuereinheit ist dann ferner dazu ausgebildet, anhand der mittels der Sensoren erfassten Druck- und Temperaturwerte die optimale Zündspannung und optional die Zündfrequenz aus der im Speicher abgelegten Lookup-Tabelle auszulesen und die DBDI-Quelle mit dieser Zündspannung und optional Zündfrequenz anzusteuern.

In einer besonders bevorzugten Ausführungsform des erfindungsgemäßen Ionenmobilitätsspektrometers ist die Lichtquelle zum Bestrahlen der DBDI-Quelle mit Licht einer Wellenlänge von 405nm und/oder einer optischen Leistung von weniger als 100mW ausgebildet. Die Erfinder haben experimentell gefunden, dass zur Zündspannungserniedrigung an einer DBDI-Quelle zum Zünden eines Niedertemperaturplasmas in getrockneter Luft insbesondere Wellenlängen von 405nm geeignet sind. Derartige Lichtquellen werden beispielsweise auch als Laserdiode in Blu-Ray-Playern oder als UV-LED zur Härtung von Harzen in 3D-Druckern verwendet und sind daher vorteilhaft in kommerzieller Form vielfältig erhältlich. In einer ebenfalls bevorzugten Ausführungsform ist die Lichtquelle zum Bestrahlen der DBDI-Quelle mit Licht eine UV-LED oder blaue LED mit einer Wellenlänge von 365 nm, 385 nm, 395 nm oder 450 nm , wie sie z.B. für die Kleberaushärtung genutzt werden. Besonders vorteilhaft ist bei der Verwendung solcher Quellen mit eingeengtem Spektralbereich zum Zünden nur eine geringe optische Gesamtstrahlungsleistung kleiner 100 mW erforderlich, insbesondere im Vergleich zu breitbandigeren Quellen, wie z.B. Quarzlampen. Zudem sind die oben genannten Laserdioden oder UV-LEDs in Halbleitertechnologie gefertigt und weisen daher vorteilhaft eine geringere elektrische Verlustleistung auf, was für mobile Geräte einen geringeren Energieverbrauch und damit eine längere Einsatzzeit bedeutet.

In dem erfindungsgemäßen Ionenmobilitätsspektrometer ist der Driftraum bevorzugt zylindrisch und ferner bevorzugt zum Transport von Ionen von einem Schaltgitter, bevorzugt bestehend aus mindestens einer Gitterelektrode, zu einem Ionendetektor ausgebildet. Der Driftraum ist bevorzugt ferner so ausgebildet, dass der Ionenstrom darin entgegen einer axialen Driftgasströmung erfolgt. Die Driftgasströmung erfolgt mithin aus Richtung des Ionendetektors in Richtung des Schaltgitters. Die Driftgeschwindigkeit hängt dabei sowohl von der Einwirkung eines elektrischen Feldes zwischen Schaltgitter und Ionendetektor als auch von der Wechselwirkung der Ionen mit der Driftgasströmung ab. Bevorzugt stellt sich bei einem gegebenen elektrischen Feld und einer gegebenen Driftgasströmung für einen bestimmten Ionen-Typ eine konstante Driftgeschwindigkeit ein, so dass keine makroskopische Beschleunigung der Ionen in dem Driftraum erfolgt. Die axiale Richtung bezieht sich im Rahmen der vorliegenden Offenbarung auf die Höhe des zylindrischen Driftraums.

In dem erfindungsgemäßen Ionenmobilitätsspektrometer ist der Reaktionsraum bevorzugt ebenfalls zylindrisch und in axialer Richtung an den Driftraum angrenzend ausgebildet. Die axiale Richtung bezieht sich dabei auf die Höhe des zylindrischen Reaktionsraums und ist bevorzugt identisch zu der axialen Richtung des zylindrischen Driftraums. Der Reaktionsraum weist einen bevorzugt zum Schaltgitter benachbarten Probengaseinlass zum Einleiten des Probengases auf. Dabei ist der Probengaseinlass derart ausgebildet, dass sich das Probengas mit dem Driftgas vermischt. Der Probengaseinlass befindet sich dabei auf einer dem Driftraum abgewandten Seite benachbart zu dem Schaltgitter. An dem gegenüberliegenden Ende des Reaktionsraums weist dieser einen Gasauslass zum Ableiten von Driftgas und von Probengas, sprich der Mischung dieser beiden Gase, auf. Ferner ist an dem Gasauslass des erfindungsgemäßen Ionenmobilitätsspektrometers die DBDI-Quelle angeordnet. Ebenfalls bevorzugt sind der Drucksensor und der Temperatursensor an oder in dem Gasauslass angeordnet.

Die DBDI-Quelle des erfindungsgemäßen Ionenmobilitätsspektrometers kann realisiert sein durch einen mit den Edelgasen Helium, Krypton oder Argon gefüllten gasdichten Glaskörper, an den ein hochfrequentes Hochspannungsfelds zum Erzeugen von Vakuum-UV-Strahlung angelegt wird. Die Vakuum-UV-Strahlung verlässt den Glaskörper über ein für den Spektralbereich durchlässiges dünnes Fenster und bewirkt die Fotoionisierung des Probengases, wobei die Elektroden durch das Glas isoliert sind und sich außerhalb des Plasmas befinden.

Ebenfalls bevorzugt werden für die DBDI-Quelle Elektroden benutzt, die vollständig durch einen Plasma-beständigen Isolator wie Korona-beständiges Polyimid oder eine Glasbeschichtung geschützt sind. Besonders bevorzugt werden Drahtelektroden verwendet, deren Drähte beispielsweise aus Wolfram gebildet und mit dünnem Glas isoliert sind. Eine solche Drahtelektrode ist in Coy et al. 2016. A Gapless Micro-Dielectric-Barrier-Discharge Ion Source for Analytical Applications. ArXiv e-prints, 2016. 1602. http://arxiv.org/abs/1602.06242 beschrieben, auf deren Inhalt hiermit vollumfänglich Bezug genommen wird. Wird eine solche Elektrode mit hochfrequenter Hochspannung beaufschlagt, bildet sich Plasma in der umgebenden Luft an den Berührungspunkten der Glasmantel. Bereits Coy et al. beobachteten dabei die Bildung von NOx-Verbindungen. Eine netzartige Anordnung derartiger Elektroden, in der sich Drähte paarweise wie beim Einzelkontakt berühren, ist für die Verwendung im erfindungsgemäßen Ionenmobilitätsspektrometer ebenfalls besonders bevorzugt.

Besonders bevorzugt weist das erfindungsgemäße Ionenmobilitätsspektrometer eine modulare DBDI-Quelle auf, welche sich einfacher in bestehende, zylindrische Aufbauten von Ionenmobilitätsspektrometern integrieren lässt, als eine in die Basisröhre vollintegrierte DBDI-Quelle. Die DBDI-Quelle ist bevorzugt als Schichtanordnung mit abwechselnden Isolator- und Elektrodenschichten realisiert, wobei die Elektroden in einer Isolator-Matrix eingebettet sind und wobei die Schichtanordnung durchbohrt ist, um die Strömung und den Transport des Probengases zu gewährleisten.

In einer bevorzugten Ausführungsform des erfindungsgemäßen Ionenmobilitätsspektrometers ist der Reaktionsraum zylindrisch ausgebildet und weist die DBDI-Quelle eine isolierende Lochscheibe auf, deren Außenumfang in einen Rand des Reaktionsraums integriert ist. Beispielsweise ist der Reaktionsraum aus einer Vielzahl ringförmiger Segmente aufgebaut und die DBDI-Quelle ist als Modul zwischen zwei solchen ringförmigen Segmenten integriert. Darüber hinaus kann die Lochscheibe jedoch auch in ein Segment des Reaktionsraums oder anderweitig in einen Außenumfang des Reaktionsraums integriert sein. Zumindest zwei gegenpolige Elektroden erstrecken sich von dem Außenumfang der isolierenden Lochscheibe in Richtung eines Innenumfangs der isolierenden Lochscheibe. Mithin erstrecken sich die Elektroden zumindest anteilig in radiale Richtung, die Elektroden erstrecken sich bevorzugt auch über den Außenumfang und/oder den Innenumfang hinaus. Zumindest eine Plasmazone ist zwischen den zumindest zwei gegenpoligen Elektroden ausgebildet.

Der Außenumfang der Lochscheibe ist bevorzugt größer als ein Innenumfang des Reaktionsraums und der Innenumfang der Lochscheibe ist bevorzugt gleich oder kleiner als der Innenumfang des Reaktionsraums. In einer Ausführungsform mit einem Innenumfang der Lochscheibe der kleiner ist, als der Innenumfang des Reaktionsraums sind die Elektroden bevorzugt in einer mittleren Isolator-Schicht angeordnet, um nicht mit Luft in Berührung zu kommen. Ferner bevorzugt sind die Elektroden entlang oder nahe des Innenumfangs freigelegt, wobei gegenpolige Elektroden voneinander durch eine mittlere isolierende Schicht getrennt sind, so dass sich zwischen diesen eine Plasmazone aufbaut. Der verengte kleinere Innenumfang der Lochscheibe bewirkt vorteilhaft einen raschen Abtransport des sich in der Plasmazone bildenden Ozons und der Stickoxide. Bei hochfrequenter Zündspannung ist wegen der gebildeten Kondensatoranordnung auf eine geringe Elektrodenfläche zu achten, bevorzugt sind die Elektroden als dünne Ringe oder schmale parallele Sektoren ausgebildet und bevorzugt über dünne, auseinanderlaufende Anschlussdrähte nach außen geführt.

In einer besonders bevorzugten Ausführungsform des erfindungsgemäßen Ionenmobilitätsspektrometers weist die DBDI-Quelle mit isolierender Lochscheibe zumindest zwei isolierende Speichen auf, die sich jeweils über eine zentrale Öffnung der isolierenden Lochscheibe erstrecken. Die isolierenden Speichen kreuzen einander innerhalb der zentralen Öffnung und tragen jeweils zumindest eine der paarweise gegenpoligen Elektroden. Beispielsweise ist pro Speiche eine Elektrode angeordnet. Alternativ sind auf den zwei, in axialer Richtung in verschiedene Richtung weisenden Oberflächen der Speiche jeweils eine Elektrode angeordnet.

Eine solche DBDI-Quelle lässt sich vorteilhaft in eine herkömmliche zylindrische Randstruktur eines Ionenmobilitätsspektrometers als spannungsisolierendes Segment integrieren und weist ferner eine stabilisierende Skelettstruktur in Gestalt von Speichen bzw. Stegen auf, die sich in der Zylindermitte des Reaktionsraums kreuzen und als Träger der Elektroden der DBD-Quelle dienen. Besonders bevorzugt weist die DBDI-Quelle ein zylindrisches skelettiertes isolierendes Randsegment (Lochscheibe) mit Speichen beziehungsweise Stegen aus demselben isolierenden Material auf. Die DBDI-Quelle ist bevorzugt in einem Herstellungsprozess, besonders bevorzugt durch Ausfräsen oder andere spanende Fertigungsverfahren, oder in einem additiven Verfahren, besonders bevorzugt durch 3D-Druck gefertigt. Bevorzugt sind die isolierende Lochscheibe und die isolierenden Speichen monolithisch und/oder aus einem/r elektrisch isolierenden Glas, Kunststoff, Quarz oder Keramik gebildet.

Die Verwendung von Speichen als Träger der Elektroden und deren Kreuzen auf der Symmetrieachse des Reaktionsraums ermöglicht eine mittige Ausbildung einer Plasmazone, die vorteilhaft vom Probengasstrom (gegebenenfalls vermischt mit Driftgasstrom) umströmt wird. Somit werden neutrale Ozon- und Stickoxidmoleküle sicher zum Gasausgang abtransportiert und es können in der Plasmazone gebildete Primär-Ionen effektiv mit dem neutralen Probengasstrom in Reaktion treten und eine maximale Ausbeute an Produkt-Ionen erzeugen.

Eine DBDI-Quelle gemäß dieser Ausführungsform weist mindestens zwei isolierende Speichen auf. Es können aber auch mehr als zwei Speichen sein, welche jeweils eine Elektrode aufnehmen und/oder als Stützträger stabilisierend wirken. Besonders bevorzugt verjüngen sich die Speichen in radialer Richtung der DBDI-Quelle und ermöglichen so eine hohe mechanische Stabilität sowie einen geringen gegenseitigen Luftabstand (Entladungsstrecke) der Plasmazone auf der Symmetrieachse des Reaktionsraums. Eine Verjüngung in axialer Richtung des Reaktionsraums ist in Richtung des Gasauslass ebenfalls bevorzugt, aber nicht notwendig. Die Speichen verlaufen nicht zwingend von Rand zu Rand der DBDI-Quelle. Ebenfalls bevorzugt treffen sich zwei Speichen V-förmig im Zentrum der DBDI-Quelle.

In einer ebenfalls bevorzugten Ausführungsform des erfindungsgemäßen Ionenmobilitätsspektrometers sind der Reaktionsraum und die Lochscheibe konzentrisch und kreiszylinderförmig ausgebildet. Gemäß dieser Ausführungsform sind die zumindest zwei isolierenden Speichen radial ausgerichtet und kreuzen sich auf einer Symmetrieachse von Reaktionsraum und Lochscheibe. Zumindest eine Plasmazone ist somit an dem Kreuzungspunkt der zumindest zwei isolierenden Speichen gebildet und mithin auf der gemeinsamen Symmetrieachse der Rotationssymmetrie des Reaktionsraums und der Lochscheibe. In dieser Ausführungsform sind besonders bevorzugt der Driftraum und der Reaktionsraum kreiszylinderförmig ausgebildet. Mit anderen Worten weisen der Driftraum und der Reaktionsraum eine kreisförmige Querschnittsfläche auf, wobei eine radiale Richtung dieser Kreisflächen senkrecht zu der jeweiligen axialen Richtung ist. Ebenfalls bevorzugt weist die Lochscheibe eine kreisringförmige Querschnittsfläche auf. Besonders bevorzugt sind die Querschnittsflächen von Driftraum, Reaktionsraum und Lochscheibe konzentrisch zueinander, die axialen Richtungen stimmen mithin überein. Ebenfalls bevorzugt ist der Gasauslass des Reaktionsraums mit Bezug zur axialen Richtung konzentrisch zu dem Reaktionsraum und dem Driftraum.

Ebenfalls bevorzugt wird eine Ausführungsform der DBDI-Quelle mit einer Vielzahl sich kreuzender Speichen und mit einer Vielzahl gebildeter Plasmazonen. Besonders bevorzugt weist die DBDI-Quelle dieser Ausführungsform eine Vielzahl sich kreuzender Speichen in sternförmiger Anordnung auf, der Kreuzungspunkt liegt mithin auf der Symmetrieachse. Bevorzugt lassen sich mit mehreren, nicht durchgehenden Speichen in bevorzugt symmetrischer Anordnung mit einer geradzahligen Anzahl 2N und bevorzugt gleichgroßen Sektorenwinkeln zwischen den Speichen in den Luftsektoren zumindest N Plasmazonen erzeugen.

In einer ebenfalls bevorzugten Ausführungsform weist der Reaktionsraum des Ionenmobilitätsspektrometers einen Gasauslass auf und ist die DBDI-Quelle am Gasauslass angeordnet. Gemäß dieser Ausführungsform sind die Elektroden bevorzugt nur auf einer dem Gasauslass zugewandten Seite der DBDI-Quelle angeordnet. Dies hat, insbesondere bei einer unidirektionalen Umströmung der DBDI-Quelle, den Vorteil, dass durch Ozon und Radikale gebildete neutrale NOx-Verbindungen direkt zum Gasauslass gelangen und nicht weiter ionisiert werden. Somit wird eine optimale Umströmung der DBDI-Quelle sichergestellt. Ebenfalls bevorzugt sind der Drucksensor und der Temperatursensor an oder im Gasauslass angeordnet.

Ferner bevorzugt weist jede der isolierenden Speichen der DBDI-Quelle einen Graben auf und ist eine der Elektroden der DBDI-Quelle in einem solchen Graben angeordnet. Bevorzugt ist mithin das Erzeugen von Nuten oder Gräben in der isolierenden Trägerstruktur der Speichen zur Aufnahme der Elektroden. Diese befinden sich beidseitig oder einseitig auf den Speichen, bevorzugt gasausgangsseitig. Je nach Größe der DBDI-Quelle können zum Ausbilden der Gräben verschiedene Technologien verwendet werden, wie beispielsweise Schleifen, Fräsen, Gravieren, Ätzen aber auch präzise Bearbeitungsmethoden der Mikrostrukturierung oder Halbleitertechnologie. In einem Graben ist die Elektrode bevorzugt am Grabenboden befestigt, beispielsweise durch Einpressen oder Kleben. Ebenso bevorzugt ist die Elektrode als leitfähige Beschichtung in dem Graben abgeschieden, beispielsweise durch Galvanisieren oder durch ein Verfahren der Dünnschichttechnologie. Als Elektrodenmaterial sind Metalle, metallische Legierungen sowie dünne halbleitende Materialien, beispielsweise Indiumzinnoxid (ITO) bevorzugt. Ebenfalls bevorzugt ist die Grabentiefe derart gewählt, dass eine Entladung entlang der kürzesten Luftstrecke zwischen den Elektroden nur in einem für die Plasmazone vorgesehenen Bereich erfolgt. Somit kann eine Entladung über den Grabenrand vorteilhaft vermieden werden, selbst ohne eine isolierende Elektrodenbeschichtung.

Ferner bevorzugt sind die Elektroden jedoch mit einer isolierenden Versiegelung, bevorzugt mit Harz, Klebstoff oder Silikaten, beschichtet. Somit kann eine Grabentiefe vorteilhaft geringer gewählt werden, auch ein Abscheiden der Elektroden auf planaren Speichen ist vorteilhaft möglich. Die Beschichtung schützt die Elektroden weiter vorteilhaft vor aggressiven Gasen wie Ozon oder Stickoxiden in der Umgebung der Plasmaquelle sowie den sich dort bildenden Primär-Ionen und deren Folgeprodukten. Besonders bevorzugt ist das vollständige Verschließen der Gräben, wodurch vorteilhaft auch Ablagerungen an der Oberfläche minimiert werden. Eine solche Versiegelung erfolgt über geeignete Beschichtungstechnologien. In einer besonders bevorzugten Ausführungsform des erfindungsgemäßen Ionenmobilitätsspektrometers ist die isolierende Versiegelung aus einem UV-transparenten Material und als Lichtleiter für das Licht der Lichtquelle ausgebildet. Insbesondere weist die isolierende Versiegelung eine Austrittstelle für das Licht nahe der zumindest einen Plasmazone auf. Diese Austrittstelle ist beispielsweise durch Oberflächenmodifikation der Versiegelung erzeugt.

In einer ebenfalls bevorzugten Ausführungsform werden die Speichen in radialer Richtung mit Bohrungen versehen, in welche die Elektroden eingebracht sind. Besonders bevorzugt sind pro Speiche zumindest zwei durchgehend versetze Bohrungen vorgesehen, die durch eine dünne Schicht des isolierenden Trägers von weniger als z. B. 1 mm getrennt werden. Ebenfalls ist nur eine Bohrung pro Speiche angeordnet, beispielsweise ist bei nicht durchgehenden Speichen nur eine Bohrung mit einer darin eingebrachten Elektrode vorgesehen und die Plasmazone ist zwischen gegenüberliegenden Stirnflächen zweier Speichen ausgebildet.

Ebenfalls bevorzugt weist ein Gasauslass des Reaktionsraums einen geringeren Strömungsquerschnitt auf, als der Reaktionsraum und/oder der Driftraum. Besonders bevorzugt ist der Strömungsquerschnitt des Reaktionsraums geringer als der Strömungsquerschnitt des Driftraums und ist der Strömungsquerschnitt des Gasauslasses geringer als der Strömungsquerschnitt des Reaktionsraums. Die zunehmende Verjüngung der Strömungsquerschnitte bewirkt dabei vorteilhaft eine Erhöhung der Gasströme an der DBDI-Quelle.

Besonders bevorzugt ist die DBDI-Quelle im erfindungsgemäßen Ionenmobilitätsspektrometer im Gasauslass platziert und weist der Gasauslass einen Strömungsquerschnitt von 6 mm oder weniger auf. Die Anordnung im Gasauslass bezeichnet dabei bevorzugt auch eine Anordnung der Ionisierungsquelle im Reaktionsraum nahe dem Gasauslass. Die Strömungsgeschwindigkeit des Gasstroms erhöht sich entsprechend des Verhältnisses vom Strömungsquerschnitt des Reaktionsraums zum Strömungsquerschnitt im Gasauslass. Vorteilhaft werden im erfindungsgemäßen Ionenmobilitätsspektrometer unerwünschte Reaktionsprodukte der Ionisierungsquelle somit direkt durch den Gasauslass abgeführt, wobei vorteilhaft auch eine Rückströmung oder Diffusion in den Reaktionsraum unterbunden wird. Ebenfalls besonders bevorzugt sind der Druck-und der Temperatursensor an oder im Gasauslass angeordnet.

In einer ebenfalls bevorzugten Ausführungsform des Ionenmobilitätsspektrometers weist dieses ferner zumindest eine im Reaktionsraum angeordnete Potentialquelle zum Erzeugen eines elektrischen Felds zum Abtransport von an der DBDI-Quelle gebildeten Ionen in Richtung des Driftraums auf. Diese Potentialquelle ermöglicht es vorteilhaft an der DBDI-Quelle gebildete Primär-Ionen von dieser abzutransportieren und somit eine Rekombination der Ionen an der DBDI-Quelle zu verhindern. Ebenfalls bevorzugt ist das mittlere elektrische Potential der DBDI-Quelle an das Potential des umgebenden Reaktionsraumes gekoppelt, um eine Optimierung der Bildungsrate der Produkt-Ionen zu erzielen. Besonders bevorzugt wird ein im Reaktionsraum befindlicher Feldstützring in unmittelbarer Nähe der DBDI-Quelle als Potentialquelle verwendet. Mittels der Potentialquelle und dem Feld der DBDI-Quelle als Bezugspotential ergibt sich ein axiales Feld für den Transport der Primär-Ionen in den Reaktionsraum. Zudem lässt sich mittels der Potentialquelle, die bevorzugt als Feldstützring dient, im Reaktionsraum auch ein radialer Feldanteil bezogen auf die DBDI-Quelle und somit eine Aufweitung des im Durchmesser zunächst geringen Primär-Ionenstrahls erreichen. Bevorzugt befindet sich die Potentialquelle, beispielsweise der nächstliegende Feldstützring, daher auf einem im Vergleich zum mittleren Potential der DBDI-Quelle erhöhten Potential und umschließt die DBDI-Quelle zentrisch. Somit werden vorteilhaft auch die in einer größeren radialen Entfernung an der DBDI-Quelle vorbeiströmenden neutralen Proben-Moleküle ionisiert. Vorteilhafterweise folgt im Anschluss eine Zone mit Driftgeschwindigkeitsreduzierung, die man durch einen geringen oder verschwindenden Potentialunterschied zum nächsten benachbarten Feldstützring in Richtung des Driftraums erzeugt. Somit werden die Verweilzeit der Primär-Ionen und die Wahrscheinlichkeit zur Produktionenbildung aus diesen erhöht.

Ferner bevorzugt weist das erfindungsgemäße Ionenmobilitätsspektrometer einen im Bereich des Ionendetektors angeordneten Driftgaseinlass auf. In einem zylindrischen Driftraum ist der Ionendetektor bevorzugt auf der dem Reaktionsraum abgewandten Deckfläche des Driftraums angeordnet. Zumindest ein Driftgaseinlass ist bevorzugt benachbart zu dem Detektor angeordnet, beispielsweise konzentrisch um einen kreisförmigen Detektor. Bevorzugt ist der Driftgaseinlass als eine konzentrisch um den Detektor angeordnete Ringöffnung ausgebildet, was vorteilhaft eine laminare Ausbildung der Driftströmung ermöglicht.

Ebenfalls bevorzugt weist das erfindungsgemäße Ionenmobilitätsspektrometer eine Driftgaszuführung zum steuer- oder regelbaren Zuleiten von Driftgas und eine Probengaszuführung zum steuer- oder regelbaren Zuleiten von Probengas auf. Die Zuführungen sind dabei vorzugsweise außerhalb von Reaktionsraum und Driftraum angeordnet, wobei die Probengaszuführung den Probengaseinlas über die Probengaszuleitung mit Probengas (Trägergas mit oder ohne Probenmaterial) speist und wobei die Driftgaszuführung den Driftgaseinlass mit Driftgas speist. Jede der Zuführungen kann hierfür eine oder mehrere Pumpen und/oder ein oder mehrere Ventile aufweisen sowie beispielsweise als Schläuche, Verrohrungen oder Kanäle ausgebildete Gaszuleitungen aufweisen.

Ein weiterer Aspekt der vorliegenden Erfindung betrifft ein Verfahren einer Steuereinheit zum Betrieb eines Ionenmobilitätsspektrometers mit einem Driftraum und einem Reaktionsraum. Das Verfahren dient dem Ermitteln einer Ionenmobilität eines Probengases in trockener Luft als Driftgas und weist die folgenden Verfahrensschritte auf. In einem Verfahrensschritt erfolgt ein Ermitteln eines von einem im Reaktionsraum angeordneten Drucksensor erfassten Druckwerts und eines von einem im Reaktionsraum angeordneten Temperatursensor erfassten Temperaturwerts und in einem weiteren Verfahrensschritt erfolgt das Einstellen einer Zündspannung einer im Reaktionsraum angeordneten DBDI-Quelle in Abhängigkeit des ermittelten Druckwerts und des ermittelten Temperaturwerts. Ferner erfolgt im erfindungsgemäßen Verfahren das Ansteuern einer in dem Reaktionsraum angeordneten Lichtquelle zum Bestrahlen der DBDI-Quelle mit Licht einer Wellenlänge von 240nm bis 480nm, bevorzugt mit Licht einer Wellenlänge von 280 nm bis 480 nm.

Erfindungsgemäß wird die Zündspannung der DBDI-Quelle vorteilhaft anhand von im Reaktionsraum gemessenen Druck und Temperatur auf einen minimalen Wert eingestellt. Die optimale Zündspannung ist dabei ferner bevorzugt für die verwendete DBDI-Quelle und/oder für eine feste Zündspannungsfrequenz ermittelt. Durch das Einstellen der minimalen und optimalen Zündspannung wird vorteilhaft eine ausreichende Erzeugung von Proben-Ionen sichergestellt und gleichzeitig die Bildung von NOx-Ionen weitgehend vermieden.

Die optimale Zündspannung wird ferner durch das Bestrahlen der DBDI-Quelle mit Licht in einem Wellenlängenbereich von 240 bis 480 nm, bevorzugt mit Licht einer Wellenlänge von 280 nm bis 480 nm, sichergestellt, wobei eine solche Bestrahlung der DBDI-Quelle in trockener Luft mit einem Wassergehalt von weniger als 100 ppm sowohl die Zündspannung des Plasmas erniedrigt als auch die Zeit zum Zünden des Plasmas verkürzt. Vorteilhaft ist im erwähnten Wellenlängenbereich eine optische Strahlungsleistung von weniger als 100 mW ausreichend, um die Plasmazündung signifikant zu unterstützen. Besonders bevorzugt erfolgt das Bestrahlen der DBDI-Quelle mit Wellenlängen von 365 nm, 385 nm, 395 nm, 405 nm oder 450 nm.

Das Bestrahlen erfolgt bevorzugt kurzzeitig bis zum Einsetzen der Ionisierung, welche im Driftzeitspektrum anhand der Intensitäten z.B. für die Produkt-Ionen erkennbar ist. Ein Minimalwert der Intensität oder des Integrals in einem vorgegebenen Intervall lässt sich als Kriterium zum Ausschalten der Lichtquelle verwenden und eine Unterschreitung des Minimalwertes veranlasst das erneute Einschalten der Lichtquelle. Ebenfalls bevorzugt ist ein gepulster Dauerbetrieb der Lichtquelle. Die Pulsweite ist bevorzugt an die Umgebungsbedingungen wie Feuchtigkeit, Druck sowie Temperatur an oder nahe der DBDI-Quelle angepasst.

Im erfindungsgemäßen Verfahren erfolgt bevorzugt ferner ein Empfangen eines Signalwerts von einem in dem Driftraum angeordneten Ionendetektor und ein Einstellen der Zündspannung in Abhängigkeit der Druck- und Temperaturwerte, so dass ein erster Signalwert des Ionendetektors einen ersten Grenzwert überschreitet und ein zweiter Signalwert des Ionendetektors einen zweiten Grenzwert unterschreitet. Ebenfalls bevorzugt erfolgt ein Ansteuern der Lichtquelle, so dass ein erster Signalwert des Ionendetektors einen ersten Grenzwert überschreitet. Das Ermitteln der optimalen Zündspannung erfolgt bevorzugt anhand der Signalwerte des Ionendetektors des Ionenmobilitätsspektrometers sowie durch die Steuereinheit des Ionenmobilitätsspektrometers. Die Signalwerte werden bevorzugt empirisch ausgewertet und anhand der Auswertung wird die optimale Zündspannung bevorzugt für eine bestimmte Kombination von Druck und Temperatur in einer Lookup-Tabelle abgelegt.

Wie vorstehend bereits erläutert, ist eine Unterdrückung der Ionenbildung aus NOx-Verbindungen durch eine möglichst geringe Hochspannungsamplitude für die Zündung und Aufrechterhaltung des Plasmas möglich. Gleichzeitig hängt die minimale Zündspannung des Plasmas von der Gasdichte und damit nach dem idealen Gasgesetz von der Temperatur und dem absoluten Druck des Gases ab, wobei sich diese Werte während des Betriebs ändern können. Erfindungsgemäß erfolgt die Zündung und Aufrechterhaltung des Plasmas in Abhängigkeit von Temperatur und Druck des vorbeiströmenden Gases kontrolliert über eine Steuerung, welche die optimalen Hochspannungsparameter für Amplitude und Frequenz einstellt und bei Änderung nachführt. Um die Zündung und Aufrechterhaltung des Plasmas bei minimaler Spannung zu gewährleisten, erfolgt ferner bevorzugt eine Messung der Gastemperatur und des Gasdrucks in der Umgebung der Ionisierungsquelle über entsprechende Sensoren sowie eine Messung des Driftzeitspektrums durch einen Ionendetektor.

Die Verfahrensschritte des erfindungsgemäßen Verfahrens können durch elektrische oder elektronische Bauteile oder Komponenten (Hardware) durch Firmware (ASIC) implementiert sein oder durch Ausführen eines geeigneten Programms (Software) verwirklicht werden. Ebenfalls bevorzugt wird das erfindungsgemäße Verfahren durch eine Kombination von Hardware, Firmware und/oder Software verwirklicht, beziehungsweise implementiert. Beispielsweise sind einzelne Komponenten zum Durchführen einzelner Verfahrensschritte als separat integrierter Schaltkreis ausgebildet oder auf einem gemeinsamen integrierten Schaltkreis angeordnet. Einzelne zum Durchführen einzelner Verfahrensschritte eingerichtete Komponenten sind ferner bevorzugt auf einem (flexiblen) gedruckten Schaltungsträger (FPCB/PCB), einem Tape Carrier Package (TCP) oder einem anderen Substrat angeordnet.

Die einzelnen Verfahrensschritte des erfindungsgemäßen Verfahrens sind ferner bevorzugt als ein oder mehrere Prozesse ausgebildet, die auf einem oder mehreren Prozessoren in einem oder mehreren elektronischen Rechengeräten laufen und beim Ausführen von ein oder mehreren Computerprogrammen erzeugt werden. Die Rechengeräte sind dabei bevorzugt dazu ausgebildet, mit anderen Komponenten, beispielsweise einem Speicher, einer Schnittstelle sowie ein oder mehreren Sensoren, zusammenzuarbeiten, um die hierin beschriebenen Funktionalitäten zu verwirklichen. Die Anweisungen der Computerprogramme sind dabei bevorzugt in einem Speicher abgelegt, wie beispielsweise einem RAM-Element. Die Computerprogramme können jedoch auch in einem nicht-flüchtigen Speichermedium, wie beispielsweise einer CD-ROM, einem Flash-Speicher oder dergleichen abgelegt sein.

Dem Fachmann ist ferner ersichtlich, dass die Funktionalitäten von mehreren Computern (Datenverarbeitungsgeräten) kombiniert oder in einem einzigen Gerät kombiniert sein können oder dass die Funktionalität von einem bestimmten Datenverarbeitungsgerät auf eine Vielzahl von Geräten verteilt vorliegen kann, um die Schritte des erfindungsgemäßen Verfahrens auszuführen, ohne vom beschriebenen erfindungsgemäßen Verfahren abzuweichen. Die bevorzugten Durchführungsformen des erfindungsgemäßen Verfahrens korrespondieren zu bevorzugten Ausführungsform des erfindungsgemäßen Ionenmobilitätsspektrometers.

BESCHREIBUNG DER FIGUREN

Nachfolgend werden Ausführungsbeispiele der vorliegenden Erfindung anhand der beigefügten Zeichnungen erläutert. Dabei zeigen:

Figur 1     eine schematische seitliche Schnittdarstellung eines Ionenmobilitätsspektrometers gemäß einer Ausführungsform;

Figur 2     eine schematische Blockdarstellung eines Ionenmobilitätsspektrometers gemäß einer Ausführungsform;

Figur 3     schematische Darstellungen einer dielektrisch behinderten Ionisierungsquelle, DBDI-Quelle, gemäß einer Ausführungsform in Seiten- und Frontalansicht;

Figur 4     eine schematische Seiten- und Schnittdarstellung einer dielektrisch behinderten Ionisierungsquelle, DBDI-Quelle, gemäß einer weiteren Ausführungsform;

Figur 5     eine schematische Seiten- und Schnittdarstellung einer dielektrisch behinderten Ionisierungsquelle, DBDI-Quelle, gemäß einer weiteren Ausführungsform;

Figur 6     eine schematische Seiten- und Schnittdarstellung einer dielektrisch behinderten Ionisierungsquelle, DBDI-Quelle, gemäß einer weiteren Ausführungsform;

Figur 7     eine schematische Seiten- und Schnittdarstellung einer dielektrisch behinderten Ionisierungsquelle, DBDI-Quelle, gemäß einer weiteren Ausführungsform;

Figur 8     eine schematische Seitendarstellung einer dielektrisch behinderten Ionisierungsquelle, DBDI-Quelle, gemäß einer weiteren Ausführungsform;

Figur 9     ein IMS-Spektrum negativ geladener Ionen (A) ohne und (B) mit Anwesenheit von kontaminierenden NOx-Ionen im Driftraum.

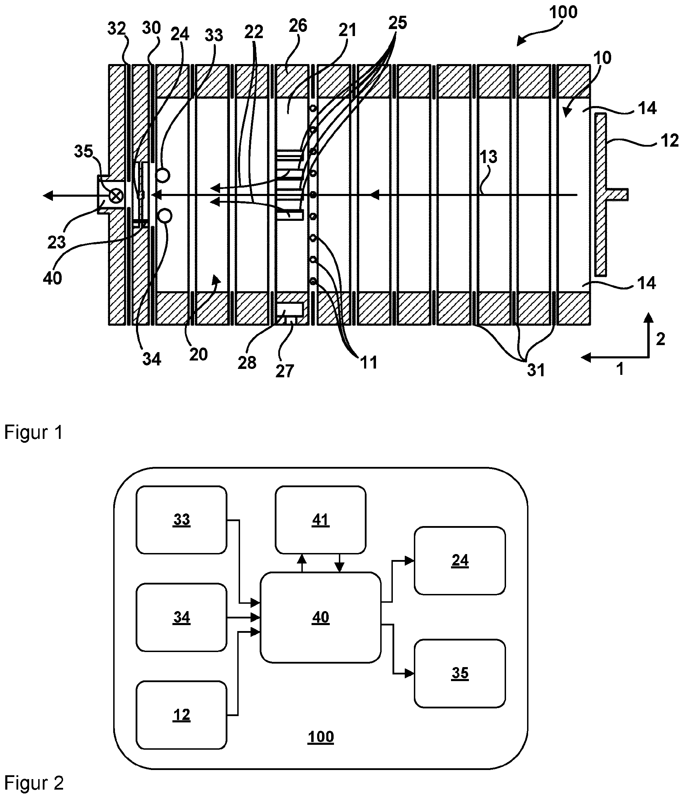

Ein Ionenmobilitätsspektrometer 100 gemäß einer Ausführungsform der vorliegenden Offenbarung ist in Figur 1 in einer schematischen Seitendarstellung gezeigt. Das Ionenmobilitätsspektrometer 100 weist dabei einen zylindrischen Driftraum 10 und einen daran in axialer Richtung 1 angrenzenden zylindrischen Reaktionsraum 20 auf. Der Driftraum 10 wird dabei auf einer dem Reaktionsraum 20 zugewandten Seite von einem Schaltgitter 11 begrenzt. Auf einer dem Schaltgitter 11 gegenüberliegenden Seite wird der Driftraum 10 von einem Ionendetektor 12 begrenzt und wird der Reaktionsraum 20 von einem Gasauslass 23 begrenzt.

Ein Driftgaseinlass 14 ist ringförmig um den Ionendetektor 12 herum angeordnet. Ein Probengaseinlass 21 ist im Reaktionsraum 20 benachbart zum Schaltgitter 11 angeordnet und weist paarweise gegenüberliegend an einem Innenumfang des Reaktionsraums 20 angeordnete Gaseinlässe 25 auf. In dem Reaktionsraum 20 ist ferner eine dielektrisch behinderte Ionisierungsquelle, DBDI-Quelle, 24 nahe des Gasauslasses 23 angeordnet.

Im Betrieb des Ionenmobilitätsspektrometer 100 wird ein Driftgas 13, insbesondere trockene Luft mit einem Wassergehalt von weniger als 100 ppm, mittels einer Driftgaszuführung (nicht dargestellt) durch den Driftgaseinlass 14 mit einem definierten Volumenstrom in den Driftraum 10 eingeleitet und besitzt hierin beispielsweise eine mittlere Strömungsgeschwindigkeit von 4 cm/s. Das Driftgas 13 durchströmt den Driftraum 10, das Schaltgitter 11 und den Reaktionsraum 20 und verlässt das Ionenmobilitätsspektrometer 100 durch den Gasauslass 23. Ferner kann im Betrieb des Ionenmobilitätsspektrometers 100 ein Probengas 22, bestehend aus in einem Trägergas enthaltenes Probenmaterial, durch einen Probengaseinlass 21 in den Reaktionsraum 20 eingeleitet werden.

Das Probengas 22 vermischt sich mit dem Driftgasstrom 13 in axialer Richtung stromabwärts von dem Probengaseinlass 21 und der Gesamtgasstrom aus Probengasstrom 22 und Driftgasstrom 22 strömt in Richtung des Gasauslass 23, wobei der Gesamtgasstrom aufgrund des geringeren Strömungsquerschnitts des Gasauslasses 23 erhöht wird. Bevor der erhöhte Gesamtgasstrom das Ionenmobilitätsspektrometer 100 durch den Gasauslass 23 verlässt, umströmt dieser die DBDI-Quelle 24, wobei je nach Fördermenge des Driftgases 13 Strömungsgeschwindigkeiten von über 50 cm/s erreicht werden. An der DBDI-Quelle 24 wird das Probenmaterial indirekt über die gebildeten Reaktant-Ionen durch eine dielektrische Barriereentladung oder direkt durch das entstehende Vakuum-UV-Licht ionisiert. Zudem kann es zu einer Ionisierung von Bestandteilen der als Driftgas 13 genutzten Luft kommen. In der Nachbarschaft der DBDI-Quelle 24 ist eine LED oder Laserdiode als Lichtquelle 35 angeordnet, mittels der DBDI-Quelle 24 direkt beleuchtet werden kann. Im eingeschalteten Zustand wird mit der LED oder Laserdiode 35 das Zünden des Plasmas bei trockenen Umgebungsbedingungen sichergestellt, insbesondere wenn sowohl das Driftgas als auch das Trägergas bzw. das Probengas trocken sind, beispielsweise mit einer Wasserkonzentration des Gesamtstroms von weniger als 100 ppm. Ebenfalls nahe der DBDI-Quelle 24 sind im Reaktionsraum 20 ein Drucksensor 33 und ein Temperatursensor 34 angeordnet. Alternativ zu der Darstellung der Figur 1 können der Drucksensor 33 und der Temperatursensor 34 besonders bevorzugt auch im Gasauslass 23 des Reaktionsraums 20 angeordnet sein.

Der Reaktionsraum 20 weist ferner eine lokale Potentialquelle 30 auf, mittels der eine elektrische Spannung gegenüber einer Referenzquelle 32 (Masse) aufgebaut werden kann. In Abhängigkeit der so erzeugten Potentialdifferenz zwischen DBDI-Quelle 24 und Potentialquelle 30 werden Ionen einer bestimmten Polarität in Richtung des Schaltgitters 11 und entgegen dem Gesamtgasstrom bewegt. Die Bewegung wird ferner durch weitere Potentialstützringe (Feldstützringe) 31 vermittelt, welche sowohl in dem Reaktionsraum 20 als auch im Driftraum 10 angeordnet sind. Dabei werden vor allem Ionen mit einem Verhältnis von Ionengeschwindigkeit und Strömungsgeschwindigkeit größer Eins entgegen den Gesamtgasstrom zum Schaltgitter 11 hinbewegt, während andere durch die hohe Strömungsgeschwindigkeit des Gesamtgasstroms zu dem Gasauslass 23 und durch diesem aus dem Ionenmobilitätsspektrometer 100 geleitet werden. Das Schaltgitter 11 erreichende Ionen werden im Driftraum durch die darin angeordneten Potentialstützringe 31 auf definierte Art und Weise gegen den Driftgasstrom 13 in Richtung des Ionendetektors 12 bewegt. Anhand der gemessenen Driftzeiten, welche die Ionen für die Strecke vom Schaltgitter 11 zu dem Ionendetektor 12 benötigen, lässt sich die Ionenmobilität der Ionen bestimmen. Es hat sich gezeigt, dass der Aufbau des Ionenmobilitätsspektrometers 100 gemäß der vorliegenden Offenbarung vorteilhaft eine Trennung von ionisiertem Probenmaterial und durch die Ionisationsquelle gebildeten neutralen Teilchen ermöglicht. Somit kann mit dem Ionenmobilitätsspektrometer 100 der vorliegenden Offenbarung ein Ionenmobilitätsspektrum ermittelt werden, dass weitgehend frei von störenden Peaks, beispielsweise von NOx-Ionen, ist.

Figur 2 zeigt eine schematische Blockdarstellung eines erfindungsgemäßen Ionenmobilitätsspektrometers 100. Dieses weist insbesondere eine Steuereinheit 40 auf, welche zum Durchführen eines erfindungsgemäßen Verfahrens zum Betrieb eines Ionenmobilitätsspektrometers 100 mit minimaler Zündspannung ausgebildet und dafür mit der DBDI-Quelle 24 verbunden ist. Die Steuereinheit 40 ist ferner mit einem im Reaktionsraum 20 angeordneten Drucksensor 33 und mit einem im Reaktionsraum 20 angeordneten Temperatursensor 34 verbunden. Die Steuereinheit 40 ist dazu ausgebildet, von dem Drucksensor 33 und dem Temperatursensor 34 einen nahe der DBDI-Quelle 24 erfassten Druckwert beziehungsweise Temperaturwert zu empfangen. Die Steuereinheit ist ferner dazu ausgebildet, anhand der empfangenen Werte und gegebenenfalls unter Zugriff auf einen Speicher 41, eine optimale und insbesondere minimale Zündspannung für eine DBDI-Quelle 24 zu ermitteln und ist ferner dazu ausgebildet, die DBDI-Quelle 24 zum Betrieb mit dieser minimalen Zündspannung anzusteuern. Die Steuereinheit ist ferner mit einem im Driftraum 10 angeordneten Ionendetektor 12 verbunden und ist dazu ausgebildet mindestens einen Signalwert von dem Ionendetektor 12 zu empfangen, wobei ein Signalwert zu einer mit dem Ionendetektor 12 detektierten Menge einer bestimmten Ionenspezies und/oder dem Integral- oder Summenwert in einem Driftzeitfenster des Driftzeitspektrums korrespondiert. Die Steuereinheit 40 ist ferner mit einer Lichtquelle 35 verbunden und ferner dazu ausgebildet, die Lichtquelle 35 anhand des zumindest einen vom Ionendetektor 12 empfangenen Signalwerts anzusteuern. Ebenfalls bevorzugt ist die Steuereinheit 40 dazu ausgebildet, die DBDI-Quelle 24 anhand von dem Ionendetektor 12 empfangener Signalwerte anzusteuern, insbesondere eine minimale Zündspannung der DBDI-Quelle 24 anhand der Signalwerte einzustellen.

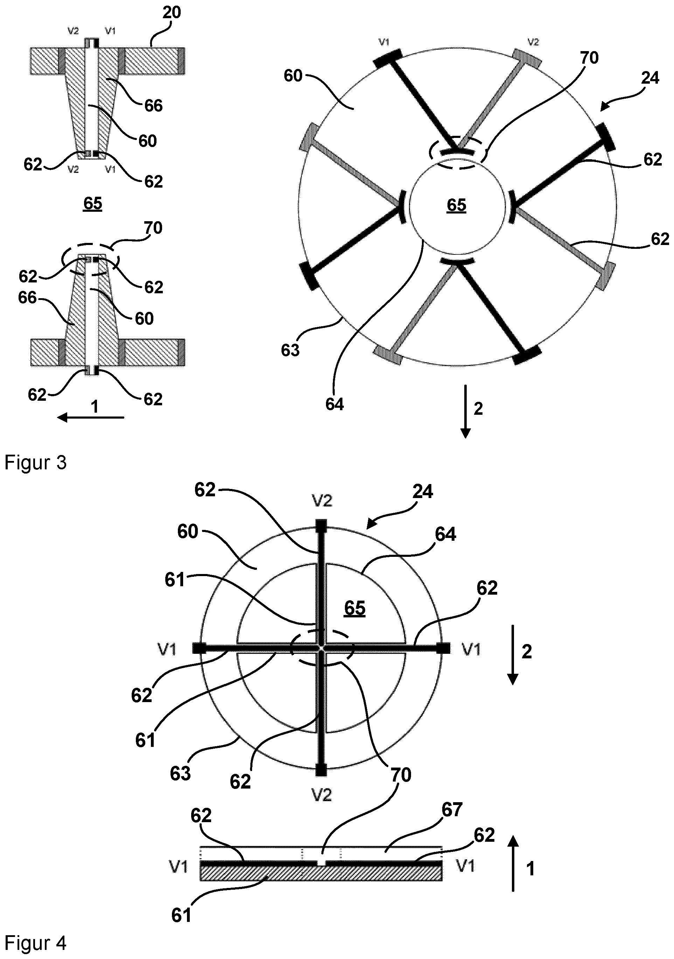

Figur 3 zeigt eine schematische Darstellung einer DBDI-Quelle 24 gemäß einer Ausführungsform in Seitenansicht (links) und in Frontalansicht (rechts). Die DBDI-Quelle 24 ist dabei in den Reaktionsraum 20 des in Figur 1 gezeigten Ionenmobilitätsspektrometers 100 integriert und weist eine isolierende Lochscheibe 60 auf, die in axialer Richtung zwischen zwei Segmente des Reaktionsraums 20 integriert ist. Stromaufwärts und stromabwärts ist die isolierende Lochscheibe 60 von Strömungsleitern 66 umgeben, welche den Strömungswiderstand der Lochscheibe 60 für den Drift- und Probengasstrom reduzieren sollen. Die isolierende Lochscheibe 60 weist auf zwei in axialer Richtung in verschiedene Richtungen weisenden Oberflächen gegenpolige Elektroden 62, insbesondere Elektroden 62 mit einem ersten Potential V1 auf einer ersten Oberfläche und Elektroden 62 mit einem Potential V2 auf einer zweiten Oberfläche. Die Elektroden 62 erstrecken sich von einem Außenumfang 63 der isolierenden Lochscheibe 60 in Richtung eines Innenumfangs 64

der isolierenden Lochscheibe 60 entlang der isolierenden Lochscheibe 60. Am oder nahe des Außenumfangs 63 sind die Elektroden 62 mit elektrischen Zuleitungen (nicht dargestellt) verbunden, welche die Elektroden 62 mit dem jeweiligen Potential V1, V2 beaufschlagen. Die Elektroden 62 erstrecken sich paarweise V-förmig und überlappen miteinander nahe des Innenumfangs 64, sprich nahe einer zentralen Öffnung 65 der isolierenden Lochscheibe. In der Frontalansicht ist lediglich das innenliegende Ende der Elektroden des Potentials V1 dargestellt, identische Enden der Elektroden des Potentials V2 überlappen jedoch auf der gegenüberliegenden Oberfläche der isolierenden Scheibe 60. Zwischen Paaren von Elektroden 62 der Potentiale V1, V2 bildet sich somit beim Anlegen einer geeigneten Zündspannung eine Plasmazone 70. Die Plasmazone erstreckt sich dabei entlang des Innenumfangs 64 in der zentralen Öffnung 65 der isolierenden Lochscheibe 60. Wird die zentrale Öffnung 65 vom Probengas 22 oder der Mischung aus Probengas 22 und Driftgas in der Driftgasströmung 13 durchströmt kommt es in den Plasmazonen 70 zur Bildung eines Niedertemperaturplasmas und somit zur Bildung von Primär-Ionen. Durch Nachfolgereaktionen entstehen somit im Reaktionsraum 20 Produkt-Ionen und gegebenenfalls NOx-Ionen.

Figur 4 zeigt eine schematische Seiten- und Schnittdarstellung einer DBDI-Quelle 24 gemäß einer weiteren Ausführungsform. Für gleiche Elemente werden in Figur 4 die gleichen Bezugszeichen verwendet, wie in Figur 3 und eine Erläuterung der DBDI-Quelle 24 der Figur 4 erfolgt insbesondere insofern sich diese DBDI-Quelle 24 von der der Figur 3 unterscheidet. Die DBDI-Quelle 24 weist ebenfalls die isolierende Lochscheibe 60 sowie monolithisch mit dieser ausgebildete isolierende Speichen 61 auf, welche sich über die zentrale Öffnung 65 erstrecken und sich im Zentrum der DBDI-Quelle 24 in der zentralen Öffnung 65 kreuzen. Die DBDI-Quelle 24 weist insbesondere zwei rechtwinklig zueinander ausgerichtete Speichen 61 auf. Auf jeder Speiche sind dabei zwei Elektroden 62 angeordnet, die sich vom Außenumfang 63 in Richtung des Innenumfangs 64 und darüber hinaus erstrecken. Die Elektroden 62 erstrecken sich bis kurz vor das Zentrum der DBDI-Quelle 24, welches sich bevorzugt auf der Rotationssymmetrieachse des Reaktionsraums 20 befindet. Die auf einer Speiche 61 ausgebildeten Elektroden 62 befinden sich jeweils auf einem ersten elektrischen Potential V1 oder einem zweiten elektrischen Potential V2. Somit sind im Zentrum der DBDI-Quelle 24 jeweils zwei Elektroden 62 rechtwinklig zueinander angeordnet, die sich auf verschiedenen elektrischen Potentialen V1, V2 befinden. Somit liegt jeweils eine Zündspannung □V=V1-V2 zwischen diesen Elektroden 62 an und es bilden sich insgesamt vier Plasmazonen 70 zwischen den rechtwinklig zueinander angeordneten Elektroden 62 aus. Die DBDI-Quelle 24 der Figur 4 ist gleich der DBDI-Quelle 24 der Figur 3 in dem Reaktionsraum 20 angeordnet, wobei die Elektroden 62 gasausgangsseitig auf Speichen 61 angeordnet sind. Somit ist eine optimale Umströmung der Plasmazonen 70 sichergestellt und gleichzeitig werden elektrisch neutrale Stickoxidverbindungen optimal zum Gasauslass 23 transportiert.

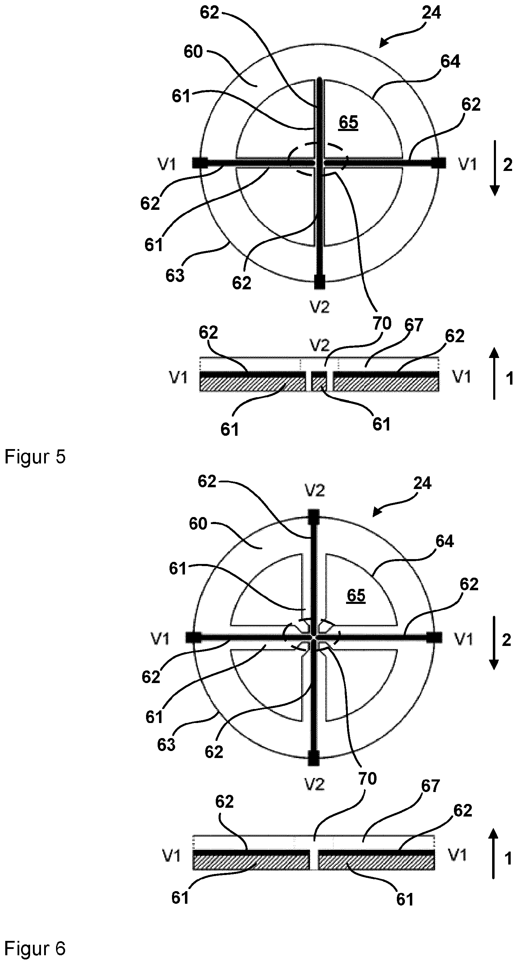

Figur 5 zeigt eine schematische Seiten- und Schnittdarstellung einer DBDI-Quelle 24 gemäß einer weiteren Ausführungsform. Für gleiche Elemente werden in Figur 5 die gleichen Bezugszeichen verwendet, wie in Figur 4 und eine Erläuterung der DBDI-Quelle 24 der Figur 5 erfolgt insbesondere insofern sich diese DBDI-Quelle 24 von der der Figur 4 unterscheidet. Die DBDI-Quelle 24 der Figur 5 weist dieselbe isolierende Lochscheibe 60 sowie dieselben isolierenden Speichen 61 auf, wie die DBDI-Quelle 24 der Figur 4, jedoch sind die Elektroden 62 anders ausgebildet. Insbesondere erstreckt sich eine Elektrode 62 auf dem Potential V2 durchgängig über die zentrale Öffnung 65, sprich über eine gesamte Speiche 61. Auf der rechtwinklig dazu angeordneten Speiche 61 sind zwei Elektroden 62 auf dem Potential V1 angeordnet, die sich jeweils von der isolierende Lochscheibe 60 bis zum Zentrum der DBDI-Quelle 24 erstrecken. Somit bilden sich zwei Plasmazonen 70 zwischen den Enden der Elektroden 62 auf Potential V1 und der Elektrode 62 auf Potential V2.

Figur 6 zeigt eine schematische Seiten- und Schnittdarstellung einer DBDI-Quelle 24 gemäß einer weiteren Ausführungsform. Für gleiche Elemente werden in Figur 6 die gleichen Bezugszeichen verwendet, wie in Figur 4 und eine Erläuterung der DBDI-Quelle 24 der Figur 6 erfolgt insbesondere insofern sich diese DBDI-Quelle 24 von der der Figur 4 unterscheidet. Die Elektrodenanordnung der DBDI-Quelle 24 der Figur 6 entspricht der Elektrodenanordnung der DBDI-Quelle 24 der Figure 4, lediglich die isolierenden Speichen 61 sind unterschiedlich ausgebildet. Insbesondere sind die isolierenden Speichen 61 nahe der isolierenden Lochscheibe 60 breiter ausgebildet, als in der Figur 4, verjüngen sich jedoch nahe dem Zentrum der DBDI-Quelle 24. Somit wird vorteilhaft eine bessere Stabilität der DBDI-Quelle 24 erreicht und dennoch geringe Luftspalte zum Ausbilden der Plasmazonen 70.

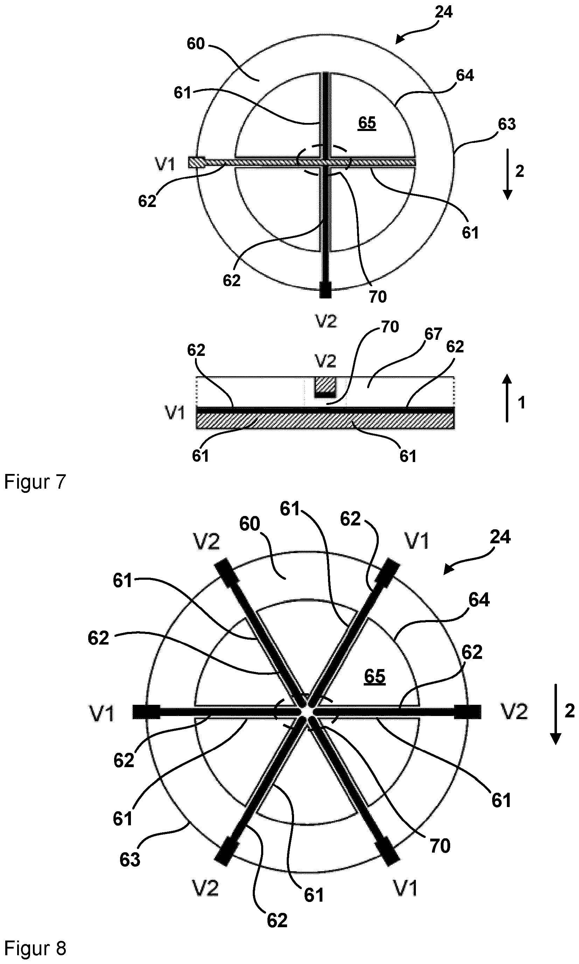

Figur 7 zeigt eine schematische Seiten- und Schnittdarstellung einer DBDI-Quelle 24 gemäß einer weiteren Ausführungsform. Für gleiche Elemente werden in Figur 7 die gleichen Bezugszeichen verwendet, wie in Figur 5 und eine Erläuterung der DBDI-Quelle 24 der Figur 7 erfolgt insbesondere insofern sich diese DBDI-Quelle 24 von der der Figur 5 unterscheidet. Die DBDI-Quelle 24 der Figur 7 weist zwei Speichen 61 auf, die sich jeweils über die gesamte zentrale Öffnung 65 erstrecken und in axialer Richtung versetzt zueinander sind. Auf jeder der Speichen 61 erstreckt sich eine Elektrode 62 über die gesamte Breite der zentralen Öffnung, wobei die beiden Elektroden 62 jeweils auf eines der Potentiale V1, V2 gelegt sind.

Figur 8 zeigt eine schematische Seitendarstellung einer DBDI-Quelle 24 gemäß einer weiteren Ausführungsform. Für gleiche Elemente werden in Figur 8 die gleichen Bezugszeichen verwendet, wie in Figur 5 und eine Erläuterung der DBDI-Quelle 24 der Figur 8 erfolgt insbesondere insofern sich diese DBDI-Quelle 24 von der der Figur 5 unterscheidet.

Die DBDI-Quelle 24 der Figur 8 weist insbesondere drei Speichen 61 auf, welche monolithisch mit der isolierenden Lochscheibe 60 ausgebildet sind und gleiche Sektorenwinkel zueinander aufweisen. Insbesondere schließen zwei in Umfangsrichtung zueinander benachbarte Speichen 61 jeweils Winkel von 60° miteinander ein. Auf jeder der Speichen 61 sind zwei Elektroden 62 angeordnet, von denen an eine das Potential V1 angelegt ist und an die andere das Potential V2 angelegt ist. Somit sind insgesamt drei Elektroden 62 auf dem elektrischen Potential V1 und drei Elektroden 62 auf dem elektrischen Potential V2, so dass beim Anlegen einer minimalen Zündspannung insgesamt sechs Plasmazonen 70 zwischen den Spitzen in Umfangsrichtung benachbarter Elektroden 62 ausgebildet werden.

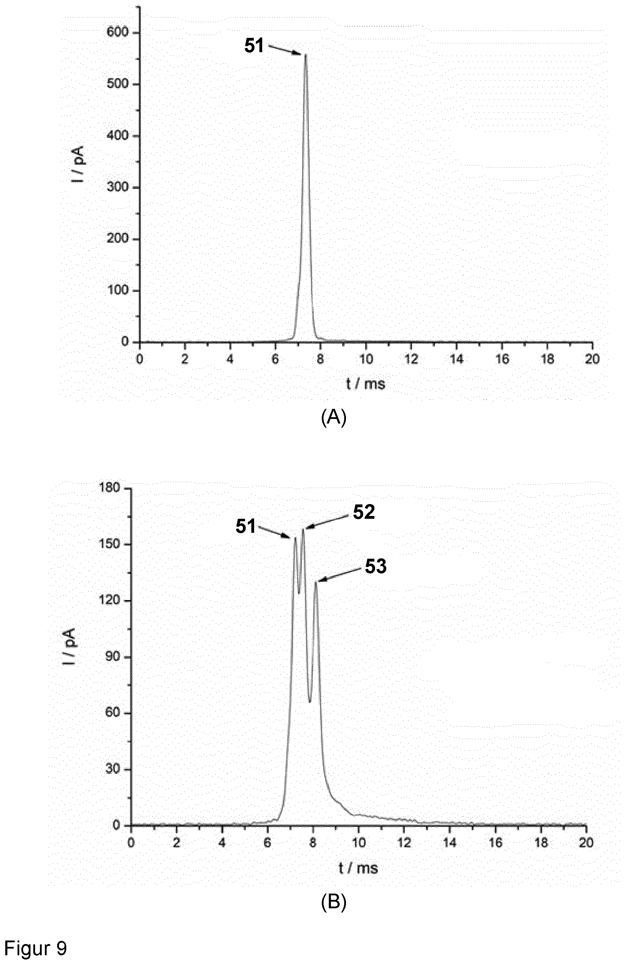

Figur 9(A) und (B) zeigen mit dem erfindungsgemäßen Ionenmobilitätsspektrometer 100 ermittelte Driftzeitspektren ohne (Fig. 9(A)) und mit (Fig. 9(B)) Kontamination des Probengases mit NOx-Ionen. In Figur 9(B) ist ein Driftzeitspektrum gezeigt, dass bei einer Zündspannung der DBDI-Quelle 24 oberhalb einer optimalen Zündspannung erzeugt wurde. Die zu hohe Zündspannung bewirkt, dass an der DBDI-Quelle 24 NOx-Ionen gebildet werden, beispielsweise $NO_2$-Ionen und/oder NOs-Ionen. In Figur 9(B) sind zwei charakteristische Peaks 52, 53 für NOx-Ionen in dem Ionenmobilitätsspektrum dargestellt. Dabei entspricht der zweite IMS-Peak 52 einem Wert einer normierten Ionenmobilität $K_0^{52}$ von 2,18 cm$^2$/Vs und der dritte IMS-Peak 53 einem Wert einer normierten Ionenmobilität $K_0^{53}$ von 2,00 cm$^2$/Vs.

Ebenfalls dargestellt ist ein erster IMS-Peak 51 eines beispielhaften ionisierten Probenmaterials mit einer Ionenmobilität $K_0^{51}$ von 2,126 cm$^2$/Vs. Wie anhand von Figur 9(B) ersichtlich, ist eine Auflösung des ersten IMS-Peaks 51 durch die IMS-Peaks 52, 53 der NOx-Ionen deutlich erschwert. Somit sollte eine Messung des IMS-Peaks 51 möglichst ohne die Anwesenheit von NOx-Ionen erfolgen. Daher ist es vorteilhaft, dass im erfindungsgemäßen Ionenmobilitätsspektrometer 100 die minimale Zündspannung anhand von den gemessenen Druck- und Temperaturwerten nahe der DBDI-Quelle 24 von der Steuereinheit 40 ermittelt und eingestellt wird. Dadurch kann das Bilden von NOx-Ionen vermieden werden und es wird folglich das Driftzeitspektrum der Figur 9(A) ausschließlich mit dem IMS-Peak 51 ermittelt.

**BEZUGSZEICHEN**

| | |
|---|---|
| 1 | axiale Richtung |
| 2 | radiale Richtung |
| 10 | Driftraum |
| 11 | Schaltgitter |
| 12 | Ionendetektor |
| 13 | Driftgasströmung |
| 14 | Driftgaseinlass |
| 20 | Reaktionsraum |
| 21 | Probengaseinlass |
| 22 | Probengas |
| 23 | Gasauslass |
| 24 | dielektrisch behinderte Ionisierungsquelle, DBDI-Quelle |
| 25 | Gaseinlässe |
| 30 | Potentialquelle |
| 31 | Feldstützring |
| 32 | Bezugspotential |
| 33 | Drucksensor |
| 34 | Temperatursensor |
| 35 | Strahlungsquelle (LED, Laser) |
| 40 | Steuereinheit |
| 41 | Speicher |
| 51 | erster IMS Peak |
| 52 | zweiter IMS Peak |
| 53 | dritter IMS Peak |
| 60 | isolierende Lochscheibe |
| 61 | isolierende Speiche |
| 62 | Elektrode |
| 63 | Außenumfang |
| 64 | Innenumfang |
| 65 | zentrale Öffnung |
| 66 | Strömungsleiter |
| 67 | isolierende Versiegelung |

70      Plasmazone
100     Ionenmobilitätsspektrometer
V1      erstes Potential
V2      zweites Potential

**Patentansprüche**

1.  Ionenmobilitätsspektrometer (100) zum Ermitteln einer Ionenmobilität eines Probengases (22) in trockener Luft als Driftgas (13) und mit einem Driftraum (10) und einem Reaktionsraum (20), das Ionenmobilitätsspektrometer (100) ferner aufweisend:

    eine im Reaktionsraum (20) angeordnete dielektrisch behinderte Ionisierungsquelle, DBDI-Quelle (24), zum Ionisieren des Probengases (22);
    einen im Reaktionsraum (20) angeordneten Drucksensor (33) und einen im Reaktionsraum (20) angeordneten Temperatursensor (34);
    eine Lichtquelle (35) zum Bestrahlen der DBDI-Quelle (24) mit Licht des Wellenlängenbereichs von 240nm bis 480nm; und
    eine mit dem Drucksensor (33), dem Temperatursensor (34), der DBDI-Quelle (24) und der Lichtquelle (35) verbundene Steuereinheit (40), **dadurch gekennzeichnet, dass** die Steuereinheit (40) dazu eingerichtet ist, eine Zündspannung der DBDI-Quelle (24) in Abhängigkeit eines durch den Drucksensor (33) ermittelten Druckwerts und eines durch den Temperatursensor (34) ermittelten Temperaturwerts einzustellen und die Lichtquelle (35) anzusteuern.

2.  Ionenmobilitätsspektrometer (100) nach Anspruch 1, aufweisend einen in dem Driftraum (10) angeordneten Ionendetektor (12), wobei die Steuereinheit (40) ferner dazu eingerichtet ist, einen Signalwert von dem Ionendetektor (12) zu empfangen und die DBDI-Quelle (24) und/oder die Lichtquelle (35) anhand des Signalwerts anzusteuern.

3.  Ionenmobilitätsspektrometer (100) nach Anspruch 2, wobei die Steuereinheit (40) ferner dazu eingerichtet ist, die Lichtquelle (35) so anzusteuern, dass ein erster Signalwert des Ionendetektors (12) einen ersten Grenzwert überschreitet.

4.  Ionenmobilitätsspektrometer (100) nach einem der vorangehenden Ansprüche, wobei die Steuereinheit (40) ferner dazu eingerichtet ist, eine Zündspannung der DBDI-Quelle (24) so einzustellen, dass bei den ermittelten Druck- und Temperaturwerten ein erster Signalwert eines Ionendetektors (12) einen ersten Grenzwert überschreitet und ein zweiter Signalwert des Ionendetektors (12) einen zweiten Grenzwert unterschreitet.

5.  Ionenmobilitätsspektrometer (100) nach einem der Ansprüche 2 bis 4, wobei die Steuereinheit (40) ferner dazu eingerichtet ist, den ersten Signalwert anhand eines Peaks eines Produkt-Ions und den zweiten Signalwert anhand eines Peaks eines NOx-Ions in einem mit dem Ionendetektor (12) ermittelten Ionenmobilitätsspektrum zu ermitteln.

6.  Ionenmobilitätsspektrometer (100) nach einem der vorangehenden Ansprüche, wobei die Lichtquelle (35) zum Bestrahlen der DBDI-Quelle (24) mit Licht einer Wellenlänge von 365 nm, 385 nm, 395 nm, 405 nm, 405nm oder 450 nm und/oder mit Licht einer optischen Leistung von weniger als 100mW ausgebildet ist.

7.  Ionenmobilitätsspektrometer (100) nach einem der vorangehenden Ansprüche, wobei der Reaktionsraum (20) zylindrisch ausgebildet ist und wobei die DBDI-Quelle (24) eine isolierende Lochscheibe (60) aufweist, deren Außenumfang (63) in einen Rand des Reaktionsraums (20) integriert ist, und wobei sich zumindest zwei gegenpolige Elektroden (62) von dem Außenumfang der isolierenden Lochscheibe (60) in Richtung eines Innenumfangs (64) der isolierenden Lochscheibe (60) erstrecken und wobei zumindest eine Plasmazone (70) zwischen den gegenpoligen Elektroden (62) ausgebildet ist.

8.  Ionenmobilitätsspektrometer (100) nach Anspruch 7, wobei die DBDI-Quelle (24) zumindest zwei isolierende Speichen (61) aufweist, die sich jeweils über eine zentrale Öffnung (65) der isolierenden Lochscheibe (60) erstrecken, die einander innerhalb der zentralen Öffnung (65) kreuzen und die zumindest eine der Elektroden (62) tragen.

9.  Ionenmobilitätsspektrometer (100) nach Anspruch 8, wobei die isolierende Lochscheibe (60) und die isolierenden Speichen (61) monolithisch und/oder aus einem/r elektrisch isolierenden Glas, Kunststoff, Quarz oder Keramik

ausgebildet sind.

10. Ionenmobilitätsspektrometer (100) nach Anspruch 8 oder 9, wobei der Reaktionsraum (20) und die Lochscheibe (60) konzentrisch kreiszylinderförmig ausgebildet sind, die isolierenden Speichen (61) radial ausgerichtet sind und sich auf einer Symmetrieachse von Reaktionsraum (20) und Lochscheibe (60) kreuzen und die zumindest eine Plasmazone (70) an dem Kreuzungspunkt der isolierenden Speichen (61) gebildet ist.

11. Ionenmobilitätsspektrometer (100) nach einem der Ansprüche 8 bis 10, wobei der Reaktionsraum (20) einen Gasauslass (23) aufweist, die DBDI-Quelle (24) am Gasauslass (23) angeordnet ist und die Elektroden (61) auf einer dem Gasauslass (23) zugewandten Seite der DBDI-Quelle (24) angeordnet sind.

12. Ionenmobilitätsspektrometer (100) nach einem der Ansprüche 8 bis 11, wobei jede der isolierenden Speichen (61) einen Graben aufweist und eine der Elektroden (62) in dem Graben angeordnet ist und/oder wobei die Elektroden (62) mit einer isolierenden Versiegelung (67), bevorzugt mit Harz, Klebstoff oder Silikaten, beschichtet sind.

13. Ionenmobilitätsspektrometer (100) nach Anspruch 12, wobei die isolierende Versiegelung (67) aus UV-transparentem Material und als Lichtleiter für das Licht der Lichtquelle (35) mit einer Austrittstelle nahe der zumindest einen Plasmazone (70) ausgebildet ist.

14. Verfahren einer Steuereinheit (40) zum Betrieb eines Ionenmobilitätsspektrometers (100), aufweisend einen Driftraum (10) und einen Reaktionsraum (20), zum Ermitteln einer Ionenmobilität eines Probengases (22) in trockener Luft als Driftgas (13), aufweisend die Verfahrensschritte:

Ermitteln eines von einem im Reaktionsraum (20) angeordneten Drucksensors (33) erfassten Druckwerts und eines von einem im Reaktionsraum (20) angeordneten Temperatursensor (34) erfassten Temperaturwerts; Einstellen einer Zündspannung einer im Reaktionsraum (20) angeordneten DBDI-Quelle (24) in Abhängigkeit des ermittelten Druckwerts und des ermittelten Temperaturwerts; und Ansteuern einer in dem Reaktionsraum angeordneten Lichtquelle (35) zum Bestrahlen der DBDI-Quelle (24) mit Licht einer Wellenlänge von 240nm bis 480nm.

15. Verfahren nach Anspruch 14, ferner aufweisend die Verfahrensschritte:

Empfangen eines Signalwerts von einem in dem Driftraum (10) angeordneten Ionendetektor (12); Einstellen der Zündspannung in Abhängigkeit der Druck- und Temperaturwerte, so dass ein erster Signalwert des Ionendetektors (12) einen ersten Grenzwert überschreitet und ein zweiter Signalwert des Ionendetektors (12) einen zweiten Grenzwert unterschreitet; und/oder Ansteuern der Lichtquelle (35), so dass der erste Signalwert des Ionendetektors (12) einen ersten Grenzwert überschreitet.

**Claims**

1. An ion mobility spectrometer (100) for determining an ion mobility of a sample gas (22) in dry air as a drift gas (13) and having a drift space (10) and a reaction space (20), the ion mobility spectrometer (100) further comprising:

a dielectrically hindered ionization source, DBDI source (24), disposed in the reaction chamber (20), for ionizing the sample gas (22); a pressure sensor (33) arranged in the reaction chamber (20) and a temperature sensor (34) arranged in the reaction chamber (20); a light source (35) for irradiating the DBDI source (24) with light of the wavelength range from 240nm to 480nm; and a control unit (40) connected to the pressure sensor (33), the temperature sensor (34), the DBDI source (24) and the light source (35), **characterized in that** the control unit (40) is adapted to set up an ignition voltage of the DBDI source (24) depending on a pressure value determined by the pressure sensor (33) and a temperature value determined by the temperature sensor (34) and to drive the light source (35).

2. Ion mobility spectrometer (100) according to claim 1, comprising an ion detector (12) arranged in the drift space (10), wherein the control unit (40) is further adapted to receive a signal value from the ion detector (12) and to control the DBDI source (24) and/or the light source (35) based on the signal value.

3. Ion mobility spectrometer (100) according to claim 2, wherein the control unit (40) is further adapted to drive the light source (35) such that a first signal value of the ion detector (12) exceeds a first threshold value.

4. Ion mobility spectrometer (100) according to any one of the preceding claims, wherein the control unit (40) is further configured to adjust an ignition voltage of the DBDI source (24) such that, for the determined pressure and temperature values, a first signal value of an ion detector (12) exceeds a first limit value and a second signal value of the ion detector (12) falls below a second limit value.

5. Ion mobility spectrometer (100) according to any one of claims 2 to 4, wherein the control unit (40) is further adapted to determine the first signal value based on a peak of a product ion and the second signal value based on a peak of a NOx ion in an ion mobility spectrum determined with the ion detector (12).

6. Ion mobility spectrometer (100) according to any one of the preceding claims, wherein the light source (35) is configured to irradiate the DBDI source (24) with light having a wavelength of 365 nm, 385 nm, 395 nm, 405 nm, 405nm or 450 nm and/or with light having an optical power of less than 100mW.

7. Ion mobility spectrometer (100) according to any one of the preceding claims, wherein the reaction chamber (20) is cylindrical in shape, and wherein the DBDI source (24) comprises an insulating apertured disk (60) having an outer periphery (63) integrated into an edge of the reaction chamber (20), and wherein at least two antipole electrodes (62) extend from the outer periphery of the insulating perforated disk (60) toward an inner periphery (64) of the insulating perforated disk (60), and wherein at least one plasma zone (70) is formed between the antipole electrodes (62).

8. Ion mobility spectrometer (100) of claim 7, wherein the DBDI source (24) comprises at least two insulating spokes (61), each extending across a central opening (65) of the insulating apertured disk (60), crossing each other within the central opening (65), and supporting at least one of the electrodes (62).

9. Ion mobility spectrometer (100) according to claim 8, wherein the insulating apertured disc (60) and the insulating spokes (61) are monolithic and/or formed of an electrically insulating glass, plastic, quartz or ceramic.

10. Ion mobility spectrometer (100) of claim 8 or 9, wherein the reaction chamber (20) and the perforated disk (60) are concentrically circular-cylindrical in shape, the insulating spokes (61) are radially aligned and intersect on an axis of symmetry of the reaction chamber (20) and the perforated disk (60), and the at least one plasma zone (70) is formed at the intersection of the insulating spokes (61).

11. Ion mobility spectrometer (100) according to any one of claims 8 to 10, wherein the reaction chamber (20) comprises a gas outlet (23), the DBDI source (24) is arranged at the gas outlet (23), and the electrodes (61) are arranged on a side of the DBDI source (24) facing the gas outlet (23).

12. Ion mobility spectrometer (100) according to any one of claims 8 to 11, wherein each of said insulating spokes (61) comprises a trench and one of said electrodes (62) is disposed in said trench and/or wherein said electrodes (62) are coated with an insulating sealant (67), preferably resin, adhesive or silicates.

13. Ion mobility spectrometer (100) according to claim 12, wherein the insulating seal (67) is made of UV-transparent material and is formed as a light guide for the light of the light source (35) with an exit point near the at least one plasma zone (70).

14. A method of a control unit (40) for operating an ion mobility spectrometer (100) comprising a drift space (10) and a reaction space (20), for determining an ion mobility of a sample gas (22) in dry air as a drift gas (13), comprising the steps of:

determining a pressure value detected by a pressure sensor (33) arranged in the reaction chamber (20) and a temperature value detected by a temperature sensor (34) arranged in the reaction chamber (20);
adjusting an ignition voltage of a DBDI source (24) disposed in the reaction chamber (20) depending on the determined pressure value and the determined temperature value; and
driving a light source (35) disposed in the reaction chamber to irradiate the DBDI source (24) with light having a wavelength of 240nm to 480nm.

15. The method of claim 14, further comprising the method steps of:

receiving a signal value from an ion detector (12) located in the drift chamber (10);
adjusting the ignition voltage as a function of the pressure and temperature values so that a first signal value of the ion detector (12) exceeds a first limit value and a second signal value of the ion detector (12) falls below a second limit value; and/or
driving the light source (35) so that the first signal value of the ion detector (12) exceeds a first limit value.

**Revendications**

1. Spectromètre de mobilité ionique (100) pour déterminer une mobilité ionique d'un échantillon gazeux (22) dans de l'air sec comme gaz de dérive (13) et comprenant un espace de dérive (10) et un espace de réaction (20), le spectromètre de mobilité ionique (100) comprenant en outre:

une source d'ionisation à barrière diélectrique, DBDI, (24), disposée dans l'espace de réaction (20), pour ioniser le gaz d'échantillonnage (22);
un capteur de pression (33) disposé dans la chambre de réaction (20) et un capteur de température (34) disposé dans la chambre de réaction (20);
une source de lumière (35) pour irradier la source DBDI (24) avec une lumière de la gamme de longueurs d'onde de 240 nm à 480 nm; et
une unité de commande (40) reliée au capteur de pression (33), au capteur de température (34), à la source DBDI (24) et à la source lumineuse (35), **caractérisé en ce que** l'unité de commande (40) est conçue pour régler une tension d'allumage de la source DBDI (24) en fonction d'une valeur de pression déterminée par le capteur de pression (33) et d'une valeur de température déterminée par le capteur de température (34) et pour commander la source lumineuse (35).

2. Spectromètre à mobilité ionique (100) selon la revendication 1, présentant un détecteur d'ions (12) disposé dans l'espace de dérive (10), l'unité de commande (40) étant en outre conçue pour recevoir une valeur de signal du détecteur d'ions (12) et pour commander la source DBDI (24) et/ou la source de lumière (35) sur la base de la valeur de signal.

3. Spectromètre de mobilité ionique (100) selon la revendication 2, dans lequel l'unité de commande (40) est en outre agencée pour commander la source de lumière (35) de telle sorte qu'une première valeur de signal du détecteur d'ions (12) dépasse une première valeur limite.

4. Spectromètre de mobilité ionique (100) selon l'une des revendications précédentes, dans lequel l'unité de commande (40) est en outre agencée pour régler une tension d'amorçage de la source DBDI (24) de telle sorte que, pour les valeurs de pression et de température déterminées, une première valeur de signal d'un détecteur d'ions (12) dépasse une première valeur limite et qu'une deuxième valeur de signal du détecteur d'ions (12) soit inférieure à une deuxième valeur limite.

5. Spectromètre de mobilité ionique (100) selon l'une des revendications 2 à 4, dans lequel l'unité de commande (40) est en outre agencée pour déterminer la première valeur de signal à l'aide d'un pic d'un ion produit et la deuxième valeur de signal à l'aide d'un pic d'un ion NOx dans un spectre de mobilité ionique déterminé avec le détecteur d'ions (12).

6. Spectromètre de mobilité ionique (100) selon l'une des revendications précédentes, dans lequel la source de lumière (35) est conçue pour irradier la source DBDI (24) avec une lumière d'une longueur d'onde de 365 nm, 385 nm, 395 nm, 405 nm, 405nm ou 450 nm et/ou avec une lumière d'une puissance optique inférieure à 100mW.

7. Spectromètre de mobilité ionique (100) selon l'une quelconque des revendications précédentes, dans lequel l'espace de réaction (20) est de forme cylindrique et dans lequel la source DBDI (24) comprend un disque perforé isolant (60) dont la périphérie extérieure (63) est intégrée dans un bord de l'espace de réaction (20), et dans lequel au moins deux électrodes (62) à polarité opposée s'étendent depuis la périphérie extérieure du disque perforé isolant (60) en direction d'une périphérie intérieure (64) du disque perforé isolant (60) et dans lequel au moins une zone de plasma (70) est formée entre les électrodes (62) à polarité opposée.

8. Spectromètre de mobilité ionique (100) selon la revendication 7, dans lequel la source DBDI (24) comprend au moins deux rayons isolants (61) qui s'étendent chacun à travers une ouverture centrale (65) du disque perforé isolant (60), qui se croisent à l'intérieur de l'ouverture centrale (65) et qui supportent au moins l'une des électrodes (62).

9. Spectromètre de mobilité ionique (100) selon la revendication 8, dans lequel le disque perforé isolant (60) et les rayons isolants (61) sont monolithiques et/ou formés d'un verre, d'une matière plastique, d'un quartz ou d'une céramique électriquement isolant(e).

10. Spectromètre de mobilité ionique (100) selon la revendication 8 ou 9, dans lequel l'espace de réaction (20) et le disque perforé (60) ont une forme de cylindre circulaire concentrique, les rayons isolants (61) sont orientés radialement et se croisent sur un axe de symétrie de l'espace de réaction (20) et du disque perforé (60) et la au moins une zone de plasma (70) est formée au point de croisement des rayons isolants (61).

11. Spectromètre de mobilité ionique (100) selon l'une quelconque des revendications 8 à 10, dans lequel l'espace de réaction (20) comprend une sortie de gaz (23), la source de DBDI (24) est disposée à la sortie de gaz (23) et les électrodes (61) sont disposées sur un côté de la source de DBDI (24) faisant face à la sortie de gaz (23).

12. Spectromètre de mobilité ionique (100) selon l'une quelconque des revendications 8 à 11, dans lequel chacun des rayons isolants (61) comprend une tranchée et l'une des électrodes (62) est disposée dans la tranchée et/ou dans lequel les électrodes (62) sont revêtues d'un scellement isolant (67), de préférence de résine, d'adhésif ou de silicates.

13. Spectromètre à mobilité ionique (100) selon la revendication 12, dans lequel le scellement isolant (67) est constitué d'un matériau transparent aux UV et est conçu comme un guide de lumière pour la lumière de la source lumineuse (35) avec un point de sortie proche de ladite au moins une zone de plasma (70).

14. Procédé d'une unité de commande (40) pour le fonctionnement d'un spectromètre de mobilité ionique (100), présentant un espace de dérive (10) et un espace de réaction (20), pour déterminer une mobilité ionique d'un gaz d'échantillon (22) dans de l'air sec comme gaz de dérive (13), présentant les étapes de procédé:

    déterminer une valeur de pression détectée par un capteur de pression (33) disposé dans la chambre de réaction (20) et une valeur de température détectée par un capteur de température (34) disposé dans la chambre de réaction (20);
    le réglage d'une tension d'allumage d'une source DBDI (24) disposée dans l'espace de réaction (20) en fonction de la valeur de pression déterminée et de la valeur de température déterminée; et
    la commande d'une source de lumière (35) disposée dans l'espace de réaction pour irradier la source DBDI (24) avec une lumière d'une longueur d'onde de 240 nm à 480 nm.

15. Procédé selon la revendication 14, comprenant en outre les étapes de procédé :

    la réception d'une valeur de signal d'un détecteur d'ions (12) disposé dans l'espace de dérive (10);
    réglage de la tension d'allumage en fonction des valeurs de pression et de température, de sorte qu'une première valeur de signal du détecteur d'ions (12) dépasse une première valeur limite et qu'une deuxième valeur de signal du détecteur d'ions (12) soit inférieure à une deuxième valeur limite ; et/ou
    commander la source de lumière (35) de telle sorte que la première valeur de signal du détecteur d'ions (12) dépasse une première valeur limite.

Figur 1

Figur 2

Figur 3

Figur 4

Figur 5

Figur 6

Figur 7

Figur 8

(A)

(B)

Figur 9

**IN DER BESCHREIBUNG AUFGEFÜHRTE DOKUMENTE**

**In der Beschreibung aufgeführte Patentdokumente**

- GB 2584334 A
- US 9953818 B2
- EP 1963835 A1